# EUROPÄISCHE PATENTSCHRIFT

(11) **EP 0 993 277 B1**
(45) Veröffentlichungstag und Bekanntmachung des Hinweises auf die Patenterteilung: **02.09.2009**
(21) Anmeldenummer: 99920652.7
(22) Anmeldetag: 07.04.1999
(51) Int. Cl.: A61B 17/34

(54) **FLEXIBLER TROKAR MIT STÜLPSCHLAUCHSYSTEM**
FLEXIBLE TROCAR WITH AN UPTURNING TUBE SYSTEM
TROCART FLEXIBLE AVEC SYSTEME DE TUBE SOUPLE A RECOUVREMENT

(30) Priorität: 07.04.1998 DE 19815598
(43) Veröffentlichungstag der Anmeldung: 19.04.2000
(73) Patentinhaber: Invendo Medical GmbH, 69469 Weinheim (DE)
(72) Erfinder: PAUKER, Fritz, D-86316 Wiffertshausen (DE); VIEBACH, Thomas, D-82282 Pischertshofen (DE); PAUKER, Robert, D-86438 Kissing (DE); WEIGLHOFER, Gerhard, D-86947 Schwabhausen (DE)
(74) Vertreter: TBK-Patent
(86) Internationale Anmeldenummer: PCT/EP1999/002476
(87) Internationale Veröffentlichungsnummer: WO 1999/051283

(56) Entgegenhaltungen:
- WO-A-93/13704
- DE-A- 2 820 239
- DE-C- 3 935 256
- DE-C- 4 222 121
- US-A- 4 366 810
- US-A- 5 176 126
- US-A- 5 179 935

## Beschreibung

Die Erfindung betrifft einen Trokar in Form eines flexiblen Zugangsrohrs zum Einführen in Körperhohlräume vorzugsweise unter Verwendung eines Stülpschlauchsystems für Diagnose und operative Eingriffe insbesondere im Dickdarmbereich.

Insbesondere bezieht sich die Erfindung auf ein flexibles Zugangsrohr, mittels dem sowohl Diagnosen beispielsweise durch Einführen eines Koloskops als auch Operationen nach der minimal invasiven Chirurgietechnik durch Einführen entsprechender chirurgischer Instrumente in das Zugangsrohr vom Anus aus durchführbar sind. Das neuartige flexible Zugangsrohr übernimmt folglich u.a. die Funktion bisher bekannter Trokare und wird daher aus Vereinfachungsgründen nachfolgend als ein "flexibler Trokar" bezeichnet.

### Hintergrund der Erfindung:

Bei dem Streben nach immer besseren und spezialisierteren Chirurgietechniken, bei denen immer kleinere Operationsstellen ausreichen und die daher den Organismus des Patienten immer weniger belasten, wird der technisch sinnvolle Anwendungsbereich der sogenannten minimal invasiven Chirurgie durch Entwicklung neuer Instrumente und Hilfsmittel immer mehr erweitert. So werden bereits Diagnosen sowie komplette Operationen an menschlichen Organen durch Einführen sogenannter Trokare als chirurgische Hilfsmittel über die Bauchdecke oder den Torax ausgeführt, über die chirurgische Instrumente sowie Optiken an das zu diagnostizierende oder zu operierende Organ heranführbar sind.

Jedoch sind derartige Hilfsmittel und Instrumente nicht in allen Fällen einsetzbar.

Um beispielsweise größere Geschwülste wie Karzinome o.ä. im Dickdarm, ausgenommen im Enddarmbereich, operativ zu entfernen, ist bisher zumeist eine Großoperation nach konventionellem Muster notwendig, d.h. der Bauch des Patienten muß geöffnet werden, um so an den Darm zu gelangen. Der Grund für diese aufwendige, den Patienten extrem belastende Operationstechnik besteht u.a. darin, daß der Darm für ein exaktes Operieren fixiert werden muß, d.h. der Darm wird an dem betreffenden Bereich aus dem Bauchraum genommen und durch geeignete Hilfsmittel eingespannt bzw. fixiert. Erst jetzt ist es dem Chirurgen möglich, die Geschwulst durch genaue Schnitte zu entfernen und anschließend den Darm wieder zu vernähen.

Es liegt auf der Hand, daß dieser Eingriff nicht nur eine aufwendige Operation darstellt, die für den Patienten sehr belastend ist und u.U. sogar ein großes Risiko, insbesondere bei älteren Patienten, in sich birgt, sondern daß hierbei auch sehr hohe Kosten anfallen, die einerseits durch den großen Operationsaufwand verursacht werden und andererseits als Folge der notwendigerweise langen Genesungsaufenthaltsdauer des Patienten im Krankenhaus entstehen.

Lediglich bis zu einer Tiefe von maximal ca. 20cm vom Anus in den Darm hinein ist es bisher möglich, Karzinome ohne die o.g. Prozedur zu entfernen. Dies geschieht mittels eines sogenannten Rektoskops. Bei einem Rektoskop handelt es sich um ein starres konusförmiges Rohrteil, das in den Anus eingeschoben wird und diesen um mehrere Zentimeter aufweitet. Damit hat der Operateur ausreichend Platz, mit Spezialwerkzeugen zu der erkrankten Stelle des Darms zu gelangen und die Operation vorzunehmen.

Ein Nachteil dieser Operationstechnik besteht jedoch darin, daß die Rektoskopie nur für operative Eingriffe geeignet ist, die im Dickdarm in einem Bereich innerhalb der ersten 20cm vom Anus aus liegen und dabei diese Operation nur von wenigen spezialisierten Chirurgen ausgeführt werden kann.
Ausgehend von dieser Problematik ist es die Aufgabe der Erfindung, eine vollkommen neuartige Vorrichtung zu schaffen, durch die sowohl Diagnose als auch operative Eingriffe im Dickdarmbereich über den Enddarm hinaus ermöglicht werden.

Diese Aufgabe wird durch eine Vorrichtung mit den Merkmalen des Anspruchs 1 gelöst. Weitere vorteilhafte Ausgestaltungen der Erfindung sind Gegenstand der übrigen Unteransprüche.

Die Erfindung geht dabei von der folgenden Überlegung aus:

Grundsätzlich ist es in der Endoskopie bereits möglich, mittels sogenannter Koloskope, wie sie beispielsweise aus der DE 28 20 239 A1 bekannt sind, den gesamten Darm eines Patienten vom Anus aus zu untersuchen. Diese Koloskope werden im wesentlichen zur visuellen Untersuchung des Darms vom Anus aus eingesetzt. Hierfür ist das Koloskop an seinem distalen Ende mit einer Beleuchtungseinrichtung sowie mit einer Optik vorzugsweise einem Kamerachip ausgestattet, der über Leitungen, die innerhalb eines Endoskop- bzw. Koloskopschafts zwischen Arbeitskanal und Schaftaußenmantel verlegt sind, mit einer Kamerasteuerung am Ende des Schafts verbunden ist. Die Kamerasteuerung ist wiederum über einen Videoprozessor mit einem externen Monitor verbunden, auf dem der behandelnde Arzt die zu untersuchenden Bereiche erkennen kann. Das in den Hohlraum einzuführende distale Ende des Schafts ist bei einigen Endoskopen in jede Richtung abkrümmbar ausgebildet und läßt sich mittels einer Handhabe, vorzugsweise über zwei Steuerräder mit Bremse am hinteren Endabschnitt des Koloskops manuell ähnlich eines Fingers abwinkeln. Des weiteren ist in der Regel der Koloskopschaft mit zumindest zwei Kanälen durchzogen, die sich an der vordersten Spitze des distalen Endes öffnen. Durch diese Kanäle kann bei Bedarf einmal Reinigungsfluid zum Säubern einer zu untersuchenden Stelle oder Co2 (Luft) zum Entfalten des Hohlraums gepreßt oder diverse Arbeitsgerätschaften beispielsweise Zangen oder Scheren zur Entnahme von Gewebeproben, Biopsienadeln, aufheizbare Schneiddrähte, Koagulationselektroden etc. eingeschoben werden, die am hinteren Ende des Koloskopschafts ebenfalls über Bedienungsdrähte oder Bowdenzüge innerhalb des inneren Kanals manuell betätigt werden können. Bei einer Gewebeprobeentnahme wird, nachdem das distale Ende die betreffende Stelle erreicht hat, beispielsweise eine Miniaturzange vom hinteren Abschnitt des Koloskopschafts aus in den Kanal eingeführt und zum distalen Ende vorgeschoben. Nach erfolgter Entnahme der Probe wird die Zange wieder zurückgezogen und aus dem Kanal entfernt, so daß mit der weiteren Untersuchung fortgefahren werden kann.

Im allgemeinen hat das Koloskop eine langgestreckte schlauchförmige Gestalt mit einem Durchmesser von ca. 9 bis 15 mm und besteht aus einem biegsamen Material, um Krümmungen des zu untersuchenden Hohlraums, beispielsweise Darmwindungen folgen zu können.

Aus dem Stand der Technik, beispielsweise gemäß der DE 42 42 291 A1, ist ebenfalls ein Endoskop dieser Gattung bekannt.

Dieses Endoskop besteht im wesentlichen aus einem Endoskopkopf oder distalen Ende, an das sich ein Endoskopschaft aus einem flexiblen biegsamen Rohrkörper anschließt und einer Bedienungseinrichtung am hinteren Ende des Endoskopschafts. Die Bedienungseinrichtung hat eine Anzahl von drehbar am Endoskopschaft gelagerten Betätigungsrädern, die über Bedienungsdrähte oder Bowdenzüge, welche innerhalb des Endoskopschafts verlegt sind, mit dem distalen Ende wirkverbunden sind. Ferner ist in einem hinteren Endabschnitt des Endoskops eine erste Antriebs- oder Vorschubeinrichtung vorgesehen, die über Antriebsräder eine Antriebskraft auf den Endoskopschaft ausübt.

Um den Endoskopschaft ist zumindest in dessen vorderem Abschnitt ein Stülpschlauch angeordnet, der durch eine zweite Antriebs- oder Vorschubeinrichtung angetrieben wird. Der Stülpschlauch besteht dabei aus einem inneren Schlauchabschnitt, der gleitfähig an der Mantelfläche des Endoskops anliegt und im Bereich des distalen Endes des Endoskops zu einem vorderen äußeren Schlauchabschnitt umgestülpt ist. Der vordere äußere Schlauchabschnitt ist ferner bis zur zweiten Antriebseinrichtung zurückgeführt und an deren Gehäuse fixiert. Im hinteren Bereich des Endoskops ist der innere Schlauchabschnitt zu einem hinteren äußeren

Schlauchabschnitt umgestülpt, der ebenfalls zur zweiten Antriebseinrichtung zugeführt und an deren Gehäuse an der zum vorderen äußeren Schlauchabschnitt gegenüberliegenden axialen Endseite fixiert ist.

Die zweite Antriebseinrichtung wirkt dabei auf den inneren Stülpschlauchabschnitt, um diesen in Axialrichtung des Endoskopschafts zu bewegen. Hierfür hat die zweite Antriebseinrichtung eine Art Manschette oder Kragen, die sich in Radialrichtung zusammenziehen und dabei reibschlüssig an den inneren Schlauchabschnitt anpressen und ferner in Axialrichtung des Endoskops hubkolbenförmig bewegen läßt. Die radial wirkende Anpreßkraft der Manschette ist dabei so groß gewählt, daß zumindest ein Teil der aufgebrachten Anpreßkraft durch eine Materialverformung des inneren Schlauchabschnitts auf die Mantelfläche des Endoskopschafts übertragen wird, sodaß der Endoskopschaft zusammen mit dem inneren Stülpschlauch angetrieben wird.

Da bei dieser alleinigen Antriebsart durch die zweite Antriebseinrichtung die Vorschubgeschwindigkeit (und Strecke) des Stülpschlauchs an seinem vorderen Stülpbereich aufgrund dessen Stülpbewegung nur halb so groß wäre, wie die des Endoskopschafts, d.h. der Schaft würde mit zunehmender Eindringtiefe aus dem Stülpschlauch teleskopförmig herausfahren, übt die eingangs genannte erste Antriebseinrichtung eine Bremskraft auf den Schaft aus, die der Vorschubkraft der zweiten Antriebseinrichtung entgegenwirkt.

Die zweite Antriebseinrichtung ist dabei zu der ersten Antriebseinrichtung synchronisiert, derart, daß im Zusammenspiel beider Antriebseinrichtungen die Bewegungsgeschwindigkeit des inneren Schlauchabschnitts ineine axiale Richtung doppelt so hoch ist wie die Bewegungsgeschwindigkeit des Endoskopschafts, wobei dieser relativ zum inneren Schaft gleitet (d.h. das distale Ende des Endoskopschafts bewegt sich mit gleicher Geschwindigkeit wie der Umstülpbereich des Stülpschlauchs).

Um die Relativgleitbewegung zwischen dem Endoskopschaft und dem Stülpschlauch zu erleichtern, sieht der Stand der Technik gemäß der DE 42 42 291 A1 ferner eine Schmiervorrichtung vor, mittels der ein Schmier- oder Gleitmittel zwischen den inneren Schlauchabschnitt und dem Endoskopschaft sowie zwischen dem inneren und äußeren Schlauchabschnitt einpreßbar ist. Hierfür hat die Schmiervorrichtung eine konusförmige Hülse, die über den Endoskopschaft gestreift ist und mit dem hinteren Stülpbereich des Stülpschlauchs, der auf die konusförmige Hülse aufreitet, dichtend zusammenwirkt. Das Schmiermittel, welches mittels einer Pumpe in den Spalt zwischen der konusförmigen Hülse und dem Endoskopschaft eingepreßt wird, breitet sich zwischen dem inneren Schlauchabschnitt und dem Endoskopschaft auf die gesamte Länge aus, wobei Überschußmengen des Schmiermittels im vorderen Umstülpbereich des Stülpschlauchs in den zu untersuchenden Hohlraum austreten.

Aus dem hausinternen Stand der Technik ist der Erfinderin schließlich ein Endoskop dieser Gattung bekannt, das eine Art Doppelstülpschlauch-System verwendet, wie es nachfolgend kurz beschrieben wird:

Dieses Stülpschlauchsystem hat einen Endoskopschaft, der in einem beidseitig gestülpten Schlauch gleitend geführt ist, der wiederum durch eine Antriebseinrichtung fortbewegbar ist, die auf den inneren Schlauchabschnitt des Stülpschlauchs einwirkt. Die Antriebseinrichtung hat zumindest ein kontinuierlich antreibendes Vorschubmittel, das radial auf den inneren Schlauchabschnitt preßbar ist, um diesen in Axialrichtung des Schafts im wesentlichen kontinuierlich zu bewegen. Dies hat den großen Vorteil, daß der kontinuierliche Vortrieb des Stülpschlauchsystems exakt steuerbar und damit beispielsweise das distale Ende eines Endoskops ortsgenau führbar ist.

Hierbei ist es vorgesehen, daß die Anpreßkraft des Vorschubmittels auf den inneren Schlauchabschnitt so gewählt ist, daß der Schaft zumindest im Bereich des Vorschubmittels im direkten Reibkontakt mit dem inneren Schlauchabschnitt ist. Das Vorschubmittel wird von einem oder mehreren Reibrädern gebildet, die gegen den inneren Schlauchabschnitt mit einer vorbestimmten oder einstellbaren Anpreßkraft vorspannbar sind, so daß zum einen ein kontinuierlicher und zum anderen ein möglichst schlupffreier Vorschub des Endoskopschafts in den Hohlraum eines Patienten gewährleistet wird.

Des weiteren hat die Antriebseinrichtung eine Vorrichtung zur Synchronisation der Schaftbewegung mit der Stülpschlauchbewegung. Diese kann ein am Schaft axial fixiertes hinteres und vorderes End- oder Klemmstück sein, an dem gleitfähig je nach Vorschubrichtung der hintere oder vordere Stülpbereich des Stülpschlauchs fest anliegt, so daß der Stülpschlauch über das hintere oder vordere Endstück eine Bremskraft entgegen der gerade vorherrschenden Vorschubkraft auf den Schaft aufbringt. Alternativ hierzu kann die Synchronisationsvorrichtung ein auf den hinteren Endabschnitt des Schafts einwirkender Rollen- oder Spindelantrieb sein, der mit dem Stülpschlauchantrieb derart synchronisiert ist, daß die Vorschubgeschwindikeit des Schafts die Hälfte der Vorschubeschwindigkeit des inneren Schlauchabschnitts beträgt.

Der Stülpschlauch kann ein Schlauchführungsteil bzw. eine Hülse aus einem steifen Material aufweisen, die über den inneren Schlauchabschnitt unter Ausbildung eines Ringspalts gezogen ist und an dessen beiden axialen Endabschnitten die freien Enden äußerer Schlauchabschnitte fixiert sind, die durch Umstülpen und Zurückführen des inneren Schlauchabschnitts an zwei axial voneinander beabstandeten Stülpbereichen gebildet werden. Diese Hülse bietet nunmehr eine Aufnahme- oder Befestigungsmöglichkeit für eine Antriebseinrichtung unmittelbar am inneren Schlauchabschnitt, so daß die äußere Abmessung des gesamten Stülpschlauchsystems kompakt bleibt und damit die Handlichkeit verbessert wird.

Es kann ferner vorgesehen sein, daß die Hülse in ihrem Mittenabschnitt eine Anzahl von vorzugsweise in gleichem Winkelabstand zueinander angeordneter Öffnungen oder Längsschlitze hat. Der Stülpschlauch bildet dabei über den inneren Schlauchabschnitt, die äußeren Schlauchabschnitte sowie die Hülse einen gegenüber der Umgebung abgedichteten Hohlraum aus, der lediglich über die Öffnungen in der Hülse einen Zugang hat. Auf diese Weise wird die Hülse konstruktiv für das Anbringen einer Antriebseinrichtung vorbereitet, deren Vorschubmittel durch die Längsschlitze mit dem inneren Schlauchabschnitt in Kontakt bringbar sind. Des weiteren ist die Hülse vorteilhafterweise mit im wesentlichen in Axialrichtung verlaufenden Nuten versehen, die sich an den Stirnflächen der Hülse öffnen. Diese Nuten erleichtern im Betrieb des Systems die zwangsläufig auftretende Verlagerung des im genannten Hohlraum befindlichen Schmiermittels durch die Hülse hindurch.

Die Antriebseinrichtung des Stülpschlauchsystems kann ein zweigeteiltes, zusammenklappbares Gehäuse haben, welches manschettenförmig um die Hülse des Stülpschlauchs legbar ist und in zusammengeklapptem Zustand mit der Hülse einen nach außen dicht verschlossenen Hohlraum bildet, im welchem Reibräder untergebracht sind, die an dem Gehäuse lagernd durch das Zusammenklappen des Gehäuses durch die Öffnungen der Hülse gegen den inneren Schlauchabschnitt preßbar sind. Hierdurch wird eine kompakte, in sich geschlossene (integrierte und nicht additive) Konstruktion des Systems erreicht, wodurch die Handhabbarkeit und Funktionalität der zusammenwirkenden Teile erhöht wird.

Bei dem intern bekannten Endoskop sind ferner die Reibräder federnd am Gehäuse gelagert, um eine entsprechend der Federkraft vorbestimmte oder auch justierbare Anpreßkraft auf den inneren Schlauchabschnitt aufzubringen und auch geringfügig variable Durchmesser des verwendeten Schafts zuzulassen . Zusätzlich können die Reibräder an ihren - Laufflächen mit einem Antirutschbelag versehen oder ausgebildet sein.

Dabei hat das Endoskop das besondere technische Merkmal, daß am Gehäuse der Antriebseinrichtung ein Anschluß für das Zuführen eines Schmiermittels ausgebildet ist, welches über die Öffnungen der Hülse in den Hohlraum des Stülpschlauchs preßbar ist. Hierdurch läßt sich zum einen der Antriebsmechanismus selbst als auch zum anderen die Relativ-Gleitbewegung der inneren und äußeren Schlauchabschnitte schmieren und damit die Reibung verringern.

Als ein weiterer Aspekt des intern bekannten Endoskops ist das Stülpschlauchsystem mit einer Schmiervorrichtung für das Einpressen von Schmiermittel in einen Ringspalt zwischen dem Schaft und dem inneren Stülpschlauchabschnitt ausgebildet, wobei zumindest eine im wesentlichen radial verlaufende Bohrung oder Perforation in der Wandung des Schafts vorgesehen ist, die sich in den Ringspalt öffnet und an einer Schmiermittelfördervorrichtung angeschlossen ist. Auf diese Weise wird bei geringem baulichen Aufwand Schmiermittel unmittelbar in den Ringspalt gefördert.

Alternativ umfaßt die Schmiervorrichtung ein hinteres Klemmstück, das auf dem Schaft fixiert ist und zumindest einen Schmiermitteleinspritzschuh hat. Dieser Schuh ragt dabei in den Ringspalt hinein und ermöglicht so ein Einpressen von Schmiermittel über eine im Schuh ausgebildete Kanüle.

Das Schmiersystem zur Zuführung von Schmiermittel zu dem Stülpschlauchsystem hat unter anderem einen oder zwei Druckbehälter zur Aufnahme je eines Schmiermittelbeutels oder Schmiermittelfaltenbalgs, der als Ein-Weg-Artikel ausgebildet ist und der über je eine Kupplung mit Zuführleitungen des Schafts und der Antriebseinrichtung fluidverbindbar ist.

Der Grundgedanke der Erfindung besteht nunmehr darin, ein bekanntes, vorzugsweise das intern bekannte Endoskop zu einem chirurgischen Hilfsmittel zur Ausführung von Diagnosen und Operationen am Dickdarm mittels der Mikrochirurgietechnik umzufunktionieren, indem der vorstehend anhand des internen Stands der Technik beschriebene Endoskopschaft durch ein erfindungsgemäß gestaltetes flexibles Zugangsrohr ersetzt wird, das nach Art eines Trokars den Zugang an die Operationsstelle für chirurgische Werkzeuge und Diagnoseinstrumente ermöglicht. Experimente haben nämlich gezeigt, daß der Dickdarm eine Elastizität besitzt, die eine Aufweitung seines inneren Durchmessers auf über 25mm zuläßt. Aufgrund dieser Ergebnisse ist es möglich, ein flexibles Zugangsrohr in Verbindung mit einem Stülpschlauchtransportsystem zu entwickeln, dessen Innendurchmesser die Einführung chirurgischer Werkzeuge und anderer Instrumente, wie beispielsweise eine Optik oder einen Koloskopschaft in Körperhohlräume, wie z.B. den Dickdarm zuläßt.

Gemäß dem Patentanspruch 1 hat das erfindungsgemäße Zugangsrohr demzufolge einen flexiblen Außenmantel sowie einen radial beabstandeten flexiblen Innenmantel, die zwischen sich einen Hohlraum zur Aufnahme von Versorgungs- und Funktionskanälen ausbilden.

Die Versorgungskanäle sind dabei vorzugsweise als Luft- bzw. CO₂-Leitungen und/oder als Spülkanäle ausgebildet, während in den Funktionskanälen Bowdenzüge verlegt oder Hydraulikflüssigkeit eingefüllt sind, um das Zugangsrohr zu betätigen sowie Kameraanschlußleitungen für am distalen Ende des Zugangsrohres angeordnete Videochips sowie Lichtleiter, vorzugsweise Glasfasern geführt sind, die eine Ausleuchtung des zu untersuchenden Hohlraums ermöglichen.

Durch den entsprechend groß bemessenen Innendurchmesser des Innenmantels und die Verlegung der Versorgungs- und Funktionskanäle in den Zwischenraum zwischen Außen- und Innenmantel wird ein ausreichend großer Zugangskanal gebildet, der vom Anus her bis zu jeder gewünschten Stelle zumindest im Dickdarm unterhalb der zweiten Sigmaschleife, ggf. darüberhinaus, reicht, aber auch in andere Körperhöhlen einführbar ist.

Mit Hilfe des erfindungsgemäßen flexiblen Zugangsrohrs ist es folglich möglich, vom Anus aus genau bis zur gewünschten Stelle im Dickdarm oder darüber hinaus vorzudringen, unabhängig von der Entfernung vom Anus aus. Damit wird quasi ein Operationskanal vergleichbar zu einem Trokar geschaffen, durch den gleichzeitig oder nacheinander die gewünschten Sonden und medizinischen Werkzeuge auf einfache Art und Weise eingeführt werden können. Das Risiko, bei der Einfühung der Operationsgeräte oder Untersuchungssonden, die Darmwand zu verletzen, wird dabei eliminiert. Die Operation kann zügiger vorgenommen werden, da beispielsweise beim Auswechseln eines Werkzeuges gegen ein anderes, kein Risiko einer Darmwandverletzung befürchtet werden muß, da diese Vorgänge nur im Inneren des Trokars geschehen, d.h. die Geräte nur mit der Innenwand des Trokars in Kontakt gelangen können, nicht jedoch mit der Darmwand selbst.

Der Trokarzwischenraum, d.h. der Zwischenraum zwischen dem Innenmantel und dem Außenmantel kann, wie vorstehend bereits angedeutet wurde, auf die vielfältigste Art und Weise genutzt werden. Es können beispielsweise zwei oder mehr Luft- oder CO₂-Kanäle darin vorgesehen werden. Dadurch kann der Darm aufgeblasen und somit gleichmäßiger entfaltet werden. Ferner können zwei oder mehrere Saug- und/oder Spülkanäle vorgesehen werden. Dadurch kann das Operationsfeld und die im Trokar integrierte Optik gespült werden, oder es kann in bestimmte Richtungen gespült werden. Darüber hinaus sind auch mehrere Beleuchtungskanäle ausbildbar, um die Sicht zu verbessern und in bestimmte Richtungen leuchten zu können.

An seinem hintersten Endabschnitt ist der erfindungsgemäße Trokar zusätzlich mit einem Trokarventil und/oder einer Dichtung ausgerüstet. Wie vorstehend bereits ausgeführt wurde, sind die Versorgungskanäle für das Zuführen von CO2 oder Luft, aber auch von Spülflüssigkeit vorgesehen. Solange kein Operationsgerät in den Trokar eingeführt ist, entweicht das CO2 und/oder die Spülflüssigkeit unkontrolliert durch den Trokarinnenraum. Um dies zu verhindern ist das Trokalventil, vorzugsweise in Form eines elektromagnetischen Klappenventils vorgesehen, das den Trokarinnenraum nach Außen verschließt. Sobald ein Operationsgerät in den Trokar eingeführt werden soll öffnet sich dieses Ventil, wobei nur geringe Leckagen auftreten. Diese können zusätzlich noch durch eine Dichtung zwischen dem Trokar und dem Operationsgerät minimiert werden.

Als besonders geeignet hat sich das genannte Klappenventil sowohl mit einteiliger als auch zweigeteilter Klappe mit oder ohne Scharnier erwiesen. Aber auch aufblasbare Ringballen oder Schläuche um den Innenumfang des Trokars sind als Ventil und gleichzeitig als Dichtung denkbar.

Schließlich können spezielle Funktionskanäle zwischen dem Außen- und Innenmantel des Trokars vorgesehen sein, die am distalen Ende des Trokars verschlossen und am hinteren Ende vorzugsweise an einer Vakuumisiereinrichtung oder einer Druckbeaufschlagunseirichtung angeschlossen sind. Insbesondere das Vakuumisieren bewirkt ein sich aneinander Pressen des Außen- und Innenmantels zumindest im Bereich dieser Funktionskanäle, womit ein Aussteifen bzw. ein Einfrieren der Haltung des flexiblen Trokars erreicht wird. Dies ist besonders während der Operationsphase wichtig, in der der Trokar nach erfolgter Einführung möglichst starr und der Darm unnachgiebig gehalten werden muß.

Alternativ zum Vakuumisierverfahren könnten die speziellen Funktionskanäle auch unter Druck gesetzt werden, um eine bestimmte zeitweilige Steifigkeit des Trokars zu erhalten. Auch wäre es denkbar, ein Fluid oder Gel in die Funktionskanäle zu spritzen, welches sich unter bestimmten Bedingungen z.B. temperaturabhängig verfestigt und wieder verflüssigt.

Die Erfindung sowie deren Funktionsweise wird nachstehend anhand bevorzugter Ausführungsbeispiele unter Bezugnahme auf die begleitenden Zeichnungen näher erläutert. Es zeigen:
Fig. 1 eine Querschnittansicht eines erfindungsgemäßen Zugangsrohrs (im nachfolgenden als flexibler Trokar bezeichnet) gemäß einem bevorzugten Ausführungsbeispiel der Erfindung;
Fig. 2(a) und (b) Querschnittansichten mehrerer Alternativen des Aufbaus eines erfindungsgemäßen flexiblen Trokars;
Fig. 3 eine Seitenansicht der Gesamtanordnung des erfindungsgemäßen flexiblen Trokars in seiner Arbeitsposition sowie dessen Stülpschlauch-Transportsystems;
Fig. 4 das erfindungsgemäßes Trokar-Stülpschlauch-System mit integrierter Trokarschmierung und Reibradantrieb gemäß dem ersten Ausführungsbeispiel der Erfindung;
Fig. 4a eine Vergrößerung der Verbindungsstelle zwischen einem Auslaßschlauch eines Schmiermittel-Druckbehälters und einem Zuführschlauch des Trokar-Stülpschlauch-Systems;
Fig. 5 das flexible Trokar-Stülpschlauch-System mit einer Trokarschmierung über ein hinteres Klemmstück gemäß einem zweiten Ausführungsbeispiel der Erfindung, wobei dieses mit einem Schmiermittel-Einspritzschuh verbunden ist;
Fig. 5a einen Längsschnitt des hinteren Trokarabschnitts in Vergrößerung zur Illustration der Schmiermittelzufuhr;
Fig. 5b einen Querschnitt des hinteren Trokarabschnitts von Fig. 5a;
Fig. 5c eine Variante des Ausführungsbeispiels gemäß Fig. 5, wonach das hintere Klemmstück ein Gleitteil hat, das durch eine Feder gegen den Stülpschlauch gedrückt wird, um so sicher zu stellen, daß während der Trokarbewegung (Ein- und
Ausfahren) kein Abstand (Schlupf) zwischen den Klemmstücken und dem Stülpschlauch entsteht;
Fig. 6 eine Stülpschlauchkonstruktion, wie sie beim Trokar-Stülpschlauch-System gemäß dem ersten und zweiten Ausführungsbeispiel Anwendung findet;
Fig. 6a eine Querschnittsansicht einer Antriebs- und Führungshülse gemäß der Schnittlinie A-A in Fig. 6;
Fig. 7 ein Trokar-Stülpschlauch-System gemäß einem dritten bevorzugten Ausführungsbeispiel der Erfindung;
Fig. 8 eine alternative Ausführungsvariante zum dritten Ausführungsbeispiel.

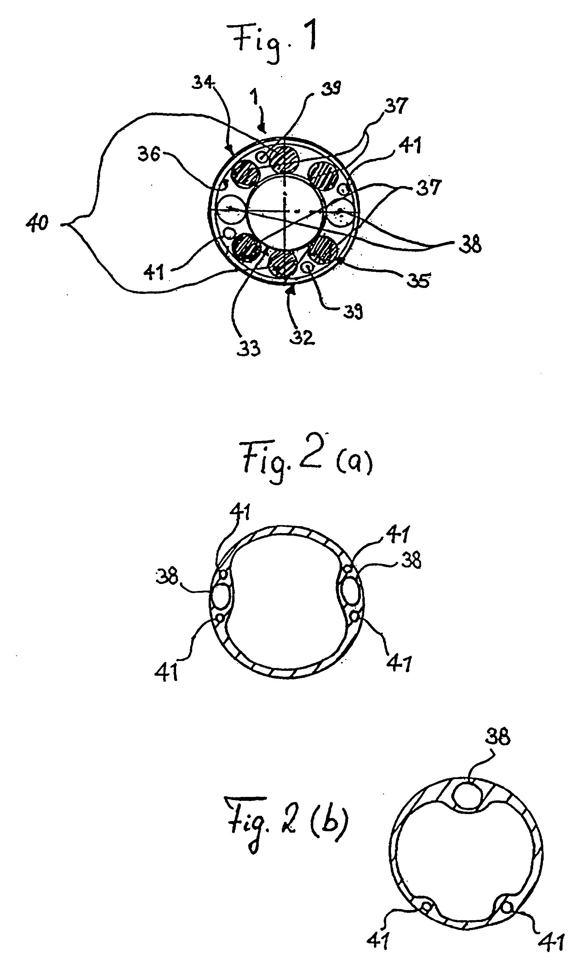

Gemäß Fig. 1, die einen Querschnitt des Trokars gemäß einem ersten Ausführungsbeispiel der Erfindung zeigt, besteht der Trokar aus einem ca. 70 bis 80 cm langen oder auch längeren hochflexiblen Trokarschaft 1, der aus einem flexiblen Außenmantel 32 und einem flexiblen Innenmantel 33 aufgebaut ist. Der Innenmantel ist dabei radial vom Außenmantel gleichmäßig beabstandet, wodurch ein Ringspalt zwischen dem Außen- und Innenmantel ausgebildet wird.

Der Außenmantel 32 hat einen Außendurchmesser von ca. 22 bis 25mm und besteht aus einer zu einer Wendel locker aufgewickelten Blattfeder vorzugsweise aus Flachbandstahl oder bandförmig verarbeitetem Kunststoff, wobei der Abstand jeweils zweier benachbarter Windungen dieser Außenwendel ca. 2 bis 3mm beträgt. Die Außenwendel ist ferner mit einer Schicht oder Haut überzogen, die vorzugsweise aus PVC oder einem anderen Kunststoff besteht und eine bestimmte Abriebfestigkeit sowie Oberflächenrauhigkeit aufweist, wodurch ein hochflexibles, geschlossenes Verbundmaterial für den Außenmantel 32 entsteht. Die Innenseite der Außenwendel kann ggf. zusätzlich mit einer dünnen Schicht vorzugsweise aus Kunststoff überzogen sein.

Der Innenmantel 33 hat einen Innendurchmesser von ca. 13-15mm und besteht aus einer Spiralwicklung vorzugsweise aus einem Rundstahldraht, der derart dicht gewickelt ist, daß im Gegensatz zur Außenwendel keine Abstände zwischen jeweils zwei benachbarten Windungen entstehen. Aufgrund der Materialwahl für die innere Spiralwicklung, nämlich Rundstahldraht, bleibt auch diese trotz der dichten Wickelweise hoch flexibel.

Der Außenmantel 32 und der Innenmantel 33 sind in diesem Ausführungsbeispiel gemäß Fig. 1 im wesentlichen über die gesamte Länge koaxial zueinander ausgerichtet, wobei der Radialabstand zwischen dem Außen- 32 und dem Innenmantel 33 durch axial voneinander beabstandete, ringscheibenförmige Stege oder auch Lochscheiben (nicht gezeigt) über die gesamte Axiallänge des flexiblen Trokars 1 beibehalten wird. Diese Stege sind an ihren inneren und äußeren radialen Rändern an dem Außen- und Innenmantel 32, 33 jeweils durch Verklemmen und ggf. zusätzlich durch Kleben fixiert und unterteilen somit den Radial- oder Zwischenspalt in mehrere Kammern. Darüber hinaus sind die einzelnen Stege jeweils durch eine Mehrzahl von Bohrungen durchdrungen, die in gleichmäßigem Winkelabstand zueinander angeordnet sind und die Kammern miteinander verbinden, um so die Durchführung von nachfolgend im Einzelnen beschriebenen Kanälen oder Röhren (für Licht, Wasser, Hydraulik, Elektrik usw.) zu ermöglichen.

Der Zwischenraum bzw. der Ringspalt zwischen dem Außen- und Innenmantel wird wie folgt genutzt:

In gleichwinkligen Abständen voneinander sind zumindest zwei, gemäß diesem Ausführungsbeispiel vier Funktionskanäle durch vier Bohrungen in den einzelnen Stegen hindurch verlegt, in die vier Bowdenzüge 37 eingezogen sind. Diese Bowdenzüge 37 sind an ihrem einen Ende an einem distalen Endabschnitt 3 des Trokarschafts 1 befestigt und an ihrem jeweils gegenüberliegenden anderen Ende an Handhaben 4, vorzugsweise Handrädchen angelenkt, um, wie dies beispielsweise bei Endoskopen üblich ist, das hochflexible distale Ende des Trokarschafts 1 in jede gewünschte Richtung abzukrümmen. Damit wird erreicht, daß man jeder vom Darm vorgegebenen Krümmung folgen und das distale Ende an jeder Stelle der Darmwand plazieren kann.

Dazwischen sind in gleichmäßigen Abständen und in symmetrischer Anordnung zwei Luft- bzw. CO₂-Kanäle 39 diametrisch gegenüberliegend angeordnet. Gleiches gilt für zwei Spül- und/oder Saugkanäle 40 und zwei optische Leitungen für je einen Kamera-Chip, die am distalen Ende des Trokars 1 angeordnet und über die Leitungen mit einem Viedoprozessor verbunden sind. Die Anordnung von zwei Kamera-Chips bietet eine hohe Ausfallsicherheit, da selbst bei Ausfall einer Kamera, die andere das Weiterführen der Operation erlaubt. Darüber hinaus sind zwei Glasfaserleitungen 41 für den Anschuß an eine Kaltlichtquelle zur Beleuchtung der Operationsstelle vorgesehen.

Die Luft- bzw. CO₂-Kanäle, die Spül-/Saugkanäle sowie die Kanäle zur Aufnahme der Lichtleiter (Glasfaserleitungen) und der Optikleitungen stellen dabei die Versorgungskanäle des Systems dar. Alternativ zu den vorstehend genannten Bowdenzügen sind natürlich auch Hydraulik- oder Pneumatikleitungen zur Betätigung, d.h. Bewegung des Trokars als Funktionskanäle denkbar.

Die Fig. 2(a) und (b) zeigen Alternativen eines Querschnittaufbaus des Trokars, die ebenso möglich sind.

In Fig. 2(a) ist der Zwischenraum zwischen dem Außenmantel 32 und dem Innenmantel 33 links und rechts dicker gewählt, so daß dort genügend Platz vorhanden ist, um in symmetrischer Anordnung je eine Kameraleitung 38 und je zwei Beleuchtungsleitungen 41 unterzubringen. Dadurch kann an den übrigen Bereichen der Zwischenraum kleiner ausgeführt sein, so daß mehr Platz im Innenmantel 33 für das Hindurchführen eines oder mehrerer Geräte vorhanden ist.

In Fig. 2(b) ist der Zwischenraum zwischen dem Außen- 32 und dem Innenmantel 33 an zumindest drei, vorzugsweise vier in gleichwinkligen Abständen voneinander beabstandeten Stellen radial vergrößert. In der gemäß Fig. 2(b) oberen radialen Aufweitung ist eine Kamera-Leitung 38 vorgesehen. An den gemäß der Fig. 2(b) beiden unteren radialen Aufweitungen sind zwei Beleuchtungsleitungen in Form von Glasfaserkabeln 41 vorgesehen. D.h. es sind nur die minimal notwendigen Funktions- und/oder Versorgungskanäle vorgesehen, um den offenen Querschnitt innerhalb des Innenmantels 33 so groß wie möglich gestalten zu können.

Wie dabei in den Fig. 2(a) und 2(b) dargestellt ist, bilden die jeweiligen radialen Aufweitungen zumindest radial nach innen vorstehende rippenförmige Vorsprünge, die sich längs des gesamten Trokars vorzugsweise durchgehend erstrecken und das einzuführende Operationsgerät oder einen Endoskopschaft gleitfähig abstützen. Hierdurch wird der Reibungswiderstand zwischen dem Operationsgerät und dem Trokar wesentlich verringert. Darüber hinaus können natürlich zusätzliche Vorsprünge unabhängig von denn vorstehend beschriebenen Aufweitungen am Innenmantel des Trokars vorgesehen sein.

Vorteilhaft ist es zudem, die Innenseite des Trokars zumindest im Bereich der radial nach innen vorstehenden Vorsprünge mit einer Teflonschicht oder einem vergleichbaren Material zu überziehen, um die Reibung weiter zu verringern.

In allen Fällen ist es denkbar, gleich mehrere Innenmäntel zur Ausbildung mehrerer Zugangsrohre vorzusehen, um für mehrere Instrumente oder Sonden je ein eigenes Zugangsrohr zur Verfügung zu haben. Es können aber auch, abhängig vom Durchmesser der einzelnen Geräte, mehrere Geräte gleichzeitig nebeneinander in ein einziges Zugangsrohr eingeführt werden.

Ferner sind alle denkbaren Varianten der Ausstattung des Zwischenraumes möglich, abhängig vom einzelnen Anwendungsfall. Es hat sich jedoch gezeigt, daß die symmetrische Anordnung der Funktions- und Versorgungskanäle in dem Ringspalt zwischen dem Außen- und Innenmantel 32, 33 des erfindungsgemäßen Trokars 1 gegenüber einer asymmetrischen Anordnungsweise vorzuziehen ist, da hierdurch ein homogenes Krümmungsverhalten des Trokars 1 etwa bei der Betätigung der Bowdenzüge erzielt wird. Das bedeutet auch, daß die Betätigungskräfte an allen Bowdenzügen für ein Abwinkeln des distalen Endes 3 des Trokars 1 in eine beliebige Richtung untereinander gleich sind und somit die Positionierbarkeit und Handhabung des Trokars 1 verbessert wird. Sofern eine asymmetrische Anordnung etwa aufgrund besonders großer Instrumente, die durch den Innenmantel 33 geführt werden sollen, notwendig ist, sollte eine solche Verteilung der Versorgungs- und Funktionskanäle in Abhängigkeit ihrer Biegesteifigkeits- Eigenschaften vorgesehen werden, daß sich auch hier eine möglichst homogene Abwinkelcharakteristik über den gesamten Umfang des Trokars 1 ergibt.

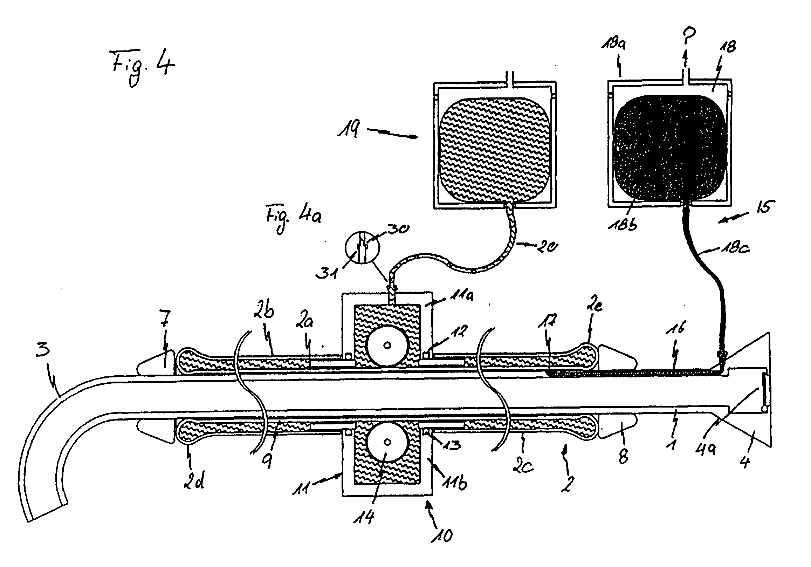

In Fig. 4 ist nunmehr der schematische Seitenriß eines Trokar-Stülpschlauch-Systems gemäß einem ersten Ausführungsbeispiel dargestellt.

Gemäß Fig. 4 besteht das Trokar-Stülpschlauch-System aus einem Trokar, d.h. aus dem Trokarschaft 1, dessen Aufbau vorstehend erläutert wurde und der über eine Länge von ca. 75 bis 100 cm mit einem Stülpschlauch 2 der Doppelstülpbauart bzw. mit beidseitiger Umstülpung ummantelt ist. Das distale Endstück 3 des Trokarschafts 1 ist für dessen Betätigung über die in der Fig. 1 mit dem Bezugszeichen 37 gekennzeichneten Bowdenzüge mit einer manuellen Betätigungseinrichtung 4 wirkverbunden, die an einem hinteren Endabschnitt des Trokarschafts 1 innerhalb eines Gehäuses untergebracht ist.

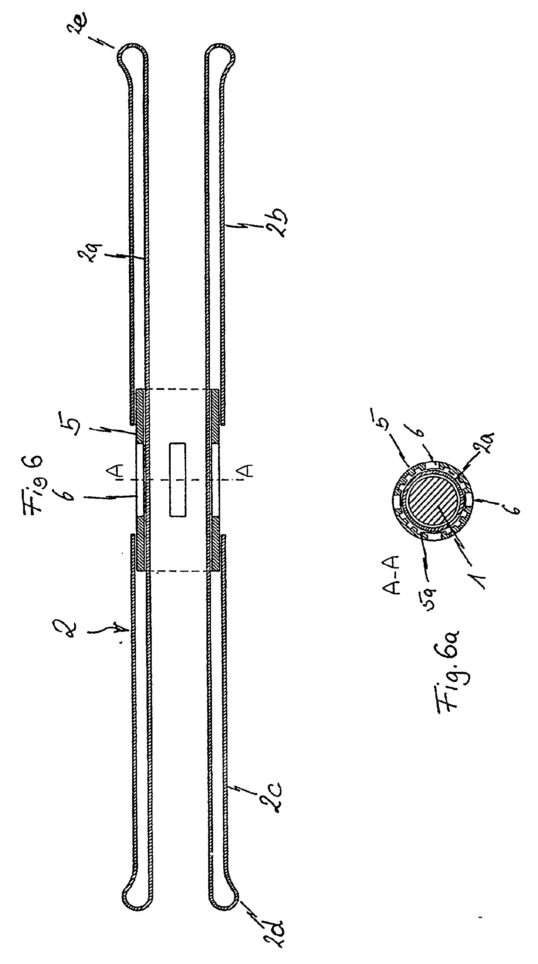

Wie insbesondere aus den Fig. 4 und 6 zu entnehmen ist, besteht der erfindungsgemäße Stülpschlauch 2 aus einem inneren Schlauchabschnitt 2a, der durch eine Antriebs- und Führungshülse bzw. ein Schlauchführungsteil 5 gleitfähig hindurchgeführt und in seinem vorderen Bereich (Umstülpbereich) zu einem vorderen äußeren Schlauchabschnitt 2b umgestülpt ist. Der vordere äußere Schlauchabschnitt 2b ist dabei zu der Antriebs- und Führungshülse (Schlauchführungsteil) 5, welche aus einem steifen Material, vorzugsweise einem Kunststoffmaterial besteht, zurückgeführt und an einem axialen Ende an der Antriebshülse 5 derart befestigt, daß diese zwischen dem inneren 2a und äußeren Schlauchabschnitt 2b zu liegen kommt.

In einem hinteren Bereich (Umstülpbereich) ist der innere Schlauchabschnitt 2a zu einem hinteren äußeren Schlauchabschnitt 2c umgestülpt, der zu der Antriebshülse 5 zurückgeführt und an einem axialen Ende der Antriebshülse 5 fixiert ist. Die Antriebshülse 5 dient zum einen als Führungselement für den inneren Schlauchabschnitt 2a, um Aufwerfungen und Falten- bzw. Schuppenbildung zu verhindern und zum anderen als Verbindungsstück des vorderen äußeren 2b und hinteren äußeren Schlauchabschnitts 2c, wobei ein Mittenbereich der Antriebshülse 5 exponiert, d.h. nicht von dem Stülpschlauch 2 überdeckt verbleibt. In diesem Mittenabschnitt weist die Antriebshülse 5 zumindest eine Öffnung, vorzugsweise einen in Axialrichtung sich erstreckenden Längsschlitz 6 vorbestimmter Breite auf. Vorliegend sind vier, im gleichen Winkelabstand zueinander angeordneter Längsschlitze 6 vorgesehen, wie insbesondere in der Fig. 6a gezeigt wird. Des weiteren zeigt die Antriebshülse 5 gemäß Fig. 6a an ihrer Innenseite eine Anzahl von durchgehenden Längsnuten 5a, die sich an den Stirnseiten der Antriebshülse 5 öffnen. Diese Längsnuten 5a können dabei entweder achsparallel oder spiralförmig gezogen sein.

Wie insbesondere aus der Fig. 4 zu erkennen ist, ist das Material, d.h. die Materialart und die Materialstärke des Stülpschlauchs 2 derart gewählt, daß sich im vorderen und hinteren Stülpbereich 2d, 2e jeweils eine wulstförmige Aufweitung infolge einer Materialanhäufung an der Umstülpung ausbildet, die im inneren Bereich zu einer vorbestimmten Einengung des Innendurchmessers des Stülpschlauchs 2 führt.

Gemäß der Fig. 4 liegt der Stülpschlauch 2 an seinem vorderen Stülpbereich 2d gleitend an einem kegelförmigen vorderen Klemmstück 7 an, das zumindest in Axialrichtung fest auf dem Trokarschaft 1 sitzt und sich in Eindringrichtung des Trokarschafts 1 konisch verjüngt. Der hintere Stülpbereich 2e des Stülpschlauchs 2 liegt ebenfalls an einem hinteren Klemmstück 8 gleitend an, das in diesem besonderen Ausführungsbeispiel genau wie das vordere Klemmstück 7 fest auf dem Trokarschaft 1 sitzt und auch eine entsprechende Form besitzt. Das hintere Klemmstück 8 verjüngt sich jedoch konisch entgegengesetzt zur Eindringrichtung des Trokars. An dieser Stelle sei jedoch darauf hingewiesen, daß zumindest das hintere Klemmstück 8 eine beliebige äußere Form aufweisen kann, da es nicht in den zu untersuchenden Hohlraum eingeführt wird.

Aus der Fig. 4 ist ferner deutlich zu entnehmen, daß sich zwischen dem inneren Schlauchabschnitt 2a des Stülpschlauchs 2 und dem Trokarschaft 1 ein schmaler Ringspalt 9 ausbildet, der axial durch die beiden Wülste in den Umstülpbereichen 2d, 2e des Stülpschlauchs 2 begrenzt wird, die sich dichtend an die äußere Mantelfläche d.h. den Außenmantel des Trokars 1 anlegen.

Um die Antriebshülse 5 ist in deren Mittenbereich eine Antriebs- oder Vorschubeinrichtung 10 angeordnet. Diese Vorschubeinrichtung 10 besteht vorliegend aus einem Gehäuse 11 vorzugsweise aus einem Kunststoff oder einer nichtrostenden Metallegierung, in welchem der nachfolgend noch beschriebene Antriebsmechanismus für den Stülpschlauch 2 gemäß dem ersten zweiten und dritten Ausführungsbeispiel der Erfindung untergebracht ist. Das Gehäuse 11 selbst besteht aus zwei schalenförmigen Gehäusehälften 11a, 11b, die an einem freien Kantenabschnitt längs der Antriebshülse 5 aufklappbar mittels eines nicht gezeigten Gelenks oder Scharniers aneinanderscharniert und an dem gegenüberliegenden freien Kantenabschnitt mittels einer ebenfalls nicht gezeigten Riegeleinrichtung verriegelbar sind. Alternativ hierzu können die Gehäusehälften 11a, 11b natürlich auch vollständig geteilt und mittels zweier Riegeleinrichtungen verbindbar sein. An den bezüglich der Antriebshülse 5 querlaufenden Seitenwandungen des Gehäuses 11 befinden sich an jeder Gehäusehälfte 11a, 11b der äußeren Querschnittsform der Antriebshülse 5 angepaßte Aussparungen, die in zusammengeklapptem und verriegeltem Zustand des Gehäuses 11 jeweils ein in sich geschlossenes Ausnehmungs- oder Öffnungsprofil entsprechend der Außenkontur der Antriebshülse 5 bilden und diese nach Außen dichtend umschließen. Die Aussparungen sowie die freien Kanten der Gehäusehälften 11a, 11b sind an ihren jeweiligen Schnitt- bzw. Kantenflächen mit Nuten 12 (nicht weiter gezeigt) versehen, in die Dichtungsstreifen 13 eingeklemmt oder geklebt sind, die sich bei Zusammenklappen der Gehäusehälften 11a, 11b dichtend an die Mantelfläche der Antriebshülse 5 anlegen, um so einen Gehäuseinnenraum dichtend abzuschließen.

Vorliegend besitzt die Antriebs- und Schlauchführungshülse 5 im Querschnitt eine kreisförmige Außenkontur entsprechend dem Kreisquerschnitt des Stülpschlauchs 2, um eine möglichst dichte und spannungsfreie Verbindung mit dem Stülpschlauch zu gewährleisten. Sie kann aber auch eine linsenförmige oder anderweitige Querschnittsform in Abhängigkeit der Konstruktion des Antriebsmechanismus aufweisen.

Dieser Antriebsmechanismus umfaßt gemäß dem ersten bevorzugten Ausführungsbeispiel der Erfindung einen nicht weiter gezeigten Antriebsmotor, der über einen Getriebezug (ebenfalls nicht dargestellt) auf eine Anzahl von Reibrädern 14 einwirkt. Diese Reibräder 14 sind an den beiden Gehäusehälften 11a, 11b jeweils derart gelagert, daß sie in zusammengeklapptem bzw. geschlossenem Zustand des Gehäuses 11 jeweils in einen Längsschlitz 6 der Antriebshülse 5 eindringen und mit einer vorbestimmten vorzugsweise über Federn einstellbaren Anpreßkraft auf dem inneren Schlauchabschnitt 2a des Stülpschlauchs 2 reibschlüssig anliegen. Vorzugsweise ist die Lauffläche jedes Antriebsrads 14 mit einem Haftbelag (Beschichtung mit Keramikteilchen oder Metallgranulat vorbestimmter Körnung) zur Erhöhung des Reibungskoeffizienten zwischen dem Stülpschlauch 2 und dem Antriebsrad 14 überzogen. Alternativ können die Reibräder 14 natürlich auch als Ganzes aus einem entsprechenden Material, beispielsweise Schleifkeramik, gefertigt sein. Die Anpreßkraft ist ferner in Abhängigkeit von dem Material und der Wandstärke des Stülpschlauchs 2 so gewählt, daß ein gleichmäßiger, im wesentlichen schlupffreier Vortrieb des Stülpschlauchs 2 über die Reibräder 14 gewährleistet ist.

Das Trokar-Stülpschlauch-System gemäß dem ersten bevorzugten Ausführungsbeispiel der Erfindung ist des weiteren mit einem Schmiersystem zur Schmierung der Relativ-Gleitbewegung zwischen dem Trokar 1 und dem inneren Schlauchabschnitt 2a, sowie zwischen dem inneren und äußeren Schlauchabschnitt 2a, 2b, 2c ausgerüstet, das nachfolgend beschrieben wird.

Dieses Schmiersystem umfaßt eine erste Schmiermittelförder- und zuführvorrichtung 15 mit einer Druckluftpumpe (nicht gezeigt) bzw. einem Druckluftanschluß, der an einen Druckbehälter 18 angeschlossen ist, um diesen mit Druckluft zu beaufschlagen bzw. zuzuführen. Dieser Druckbehälter 18 besitzt einen aufklapp- oder entfernbaren Deckel 18a, mittels dem eine Be- und Entladeöffnung verschließbar ist. Innerhalb des Druckbehälters 18 befindet sich ein entnehmbarer bzw. auswechselbarer Kunststoff- oder Gummibeutel 18b oder alternativ ein Schmiermittelfaltenbalg, der mit dem zu verwendenden Schmiermittel gefüllt ist. Dieser Beutel oder Balg 18b hat einen Auslaßschlauch 18c, der durch eine, vorzugsweise der Anschlußstelle der Pumpe gegenüberliegende Bohrung des Druckbehälters 18 geführt ist. Die Bohrung schließt dabei dichtend am Auslaßschlauch 18c ab, wofür in diesem Ausführungsbeispiel ein Dichtungskeder an der Bohrung angebracht ist. Zusätzlich oder alternativ zu dieser Dichtung legt sich der Beutel 18 dichtend an der Druckbehälterwand an, so daß die Auslaßbohrung im wesentlichen luftdicht von der Anschlußstelle der Pumpe, welche sich vorzugsweise im Deckel 18a befindet, durch den Beutel 18b abgetrennt wird.

Am freien Ende des Auslaßschlauchs 18c ist ein Kupplungsstück 30 vorgesehen, das mit einem entsprechenden Kupplungsgegenstück 31 eines Zuführschlauchs 16 des Trokar-Stülpschlauch-Systems verbindbar ist, das aus dem hinteren Endabschnitt des Trokarschafts 1 insbesondere aus dem Gehäuse der Betätigungseinrichtung 4 seitlich herausragt. Der Zuführschlauch oder Kanal 16 ist hierbei, wie in der Fig. 4 andeutungsweise gezeigt wird, innerhalb des Trokarschafts 1 d.h. in dem Ringspalt zwischen dem Außenmantel 32 und dem Innenmantel 33 des Trokars 1 bis zu einer im Außenmantel 32 des Trokarschafts 1 ausgebildeten Auslaßbohrung 17 im Bereich des Stülpschlauchs 2 weiterverlegt.

Die beiden Kupplungsstücke des Auslaß- 18c und des Zuführschlauchs 16 sind in der Fig. 4a schematisch dargestellt.

Demzufolge besitzt das eine Kupplungsstück 30 einen Kupplungsflansch mit einer Überwurfmutter oder einem Bajonettverschluß, wobei die Mündung des Auslaßschlauchs 18c durch eine Membran verschlossen ist. Das andere Kupplungsgegenstück 31 hat ein der Überwurfmutter oder dem Bajonettverschluß entsprechendes Gegenelement sowie eine nadelförmig oder mit einer Schneide ausgebildeten Kanüle als äußerstes Ende des Zuführschlauchs 16. Beim Zusammenstecken der beiden Kupplungsstücke 30, 31 durchdringt die Kanüle die Membran und stellt so eine Fluidverbindung zwischen dem Auslaßschlauch 18c und dem Zuführschlauch 16 her.

Es sei darauf hingewiesen, daß die Anordnung der Kupplung 30, 31 keinesfalls am Trokar 1 sein muß. Das eine Kupplungsstück 30 kann beispielsweise auch in der Bohrung des Druckbehälter 18 integriert sein, wobei in diesem Fall das Gegenstück 31 unmittelbar am Beutel 18b plaziert werden kann, so daß bei Einsetzten des Beutels 18b automatisch eine Verbindung zum Trokar 1 hergestellt wird. In dessen ist die erste Ausführungsvariante zu bevorzugen, da hierdurch bereits bei Zusammenstecken der beiden Schläuche 18c und 16 der Auslaßschlauch 18c über seine gesamte Länge mit Schmiermittel vorab gefüllt ist und damit eine nachträgliche Entlüftung des Schmiersystems nicht mehr erforderlich ist.

Wie ferner in der Fig. 4 gezeigt ist, durchdringt der Zuführschlauch 16 die Auslaßbohrung oder Bohrungen 17, derart, daß keine Leckage an Schmiermittel in das Innere des Trokarzwischenraums und damit in den Zugangskanal des Innenmantels auftreten kann und mündet dabei in den Ringspalt 9 zwischen dem inneren Stülpschlauchabschnitt 2a und dem Trokarschaft 1, der durch den vorderen und hinteren Stülpbereich 2d, 2e axial fluiddicht begrenzt wird. Anstelle der zumindest einen Auslaßbohrung 17 kann dabei natürlich auch eine Perforation des Außenmantels 32 des Trokarschafts 1 an der entsprechenden Stelle vorgesehen sein, wobei im ringförmigen Zwischenspalt des Trokarschafts 1 eine eigene Schmiermittelleitung 16 mit mehreren Auslässen zur Manteloberfläche vorzugsweise zwischen den Windungen der Außenwendel hindurch verlegt sein kann.

Des weiteren ist im hinteren Endbereich des Trokars gemäß der Fig. 4 ein magnetisches oder elektromagnetisches Klappenventil 4a angeordnet, das den Innenraum des Trokars nach außen verschließt. Dieses Ventil besteht im wesentlichen aus einer oder zwei Klappen, die über ein Scharnier am Trokar angelenkt und mittels eines Elektromagnete betätigbar sind. Hierdurch wird eine Leckageströmung vom distalen Ende über den Innenraum bis zum Endbereich des Trokars vollständig verhindert.

Der Förderdruck der ersten Schmiermittelfördervorrichtung 15 ist ferner so gewählt, daß der entstehende Staudruck innerhalb des Ringspalts 9 zwischen dem Trokarschaft 1 und dem inneren Schlauchabschnitt 2a, kleiner ist als der Anpreßdruck der wulstförmigen Umstülpbereiche 2d, 2e des Stülpschlauchs 2 auf den Trokarschaft 1. In diesem Fall wirken die Umstülpbereiche 2d, 2e zusammen mit dem Schaft 1 unmittelbar als Dichtungen, die eine Leckage an Schmiermittel verhindern. Des weiteren hat dies den Vorteil, daß diese Art von Dichtung vorerst nicht entlang des Trokarschafts 1 oder mit entsprechendem Anpreßdruck am vorderen Endstück 7 gleiten muß sondern systementsprechend am Trokarschaft 1 abwälzt, wodurch eine Erhöhung der notwendigen Vorschubkraft auf den inneren Schlauchabschnitt 2a zur Überwindung von Reibkräften unterbleibt. Dies wiederum führt zu einer verringerten notwendigen Anpreßkraft der Reibräder 14 auf den inneren Schlauchabschnitt 2a. Auf diese Weise wird auch gewährleistet, daß der Trokarschaft 1 innerhalb des Stülpschlauchs 2 drehbar bleibt, um so bei anormalen Darmwindungen ein Durchfahren durch den Darm möglich zu machen.

Des weiteren umfaßt das Schmiersystem eine zweite Schmiermittelförder- und Zuführvorrichtung 19, die über einen Zuführschlauch oder Kanal 20 mit dem Gehäuse 11 der Antriebseinrichtung 10 verbunden ist. Der konstruktive Aufbau der zweiten Schmiermittelförder- und Zuführvorrichtung 19 entspricht im Prinzip dem der ersten Schmiermittelförder- und Zuführvorrichtung 15 bzw. den hierzu beschriebenen Alternativen, so daß an dieser Stelle auf die entsprechenden Textstellen verwiesen werden kann.

Die zweite Schmiermittelförder- und Zuführeinrichtung 19 ist hingegen wie bereits angedeutet, an das Antriebsgehäuse 11 angeschlossen, um das geförderte Schmiermittel in den dicht verschlossenen Innenraum des Antriebsgehäuses 11 zu pressen, wobei die Kupplung unmittelbar an dem Gehäuse 11 angeordnet ist. Dieses Schmiermittel hat ferner über die Öffnungen oder Längsschlitze 6 in der Antriebshülse 5 Zugang in den Hohlraum zwischen dem inneren und äußeren Schlauchabschnitt 2a, 2b, 2c des Stülpschlauchs 2, um somit die Relativ-Gleitbewegung zwischen dem inneren und äußeren Schlauchabschnitt 2a, 2b, 2c zu schmieren. Darüber hinaus bewirkt das Schmiermittel innerhalb des Antriebsgehäuses 11 natürlich auch eine Schmierung des Antriebsmechanismus, d.h. der Lagerung der Reibräder 14 selbst.

Zur Funktions- und Betriebsweise des Trokar-Stülpschlauchsystems gemäß dem ersten Ausführungsbeispiel der Erfindung wird folgendes ausgeführt:

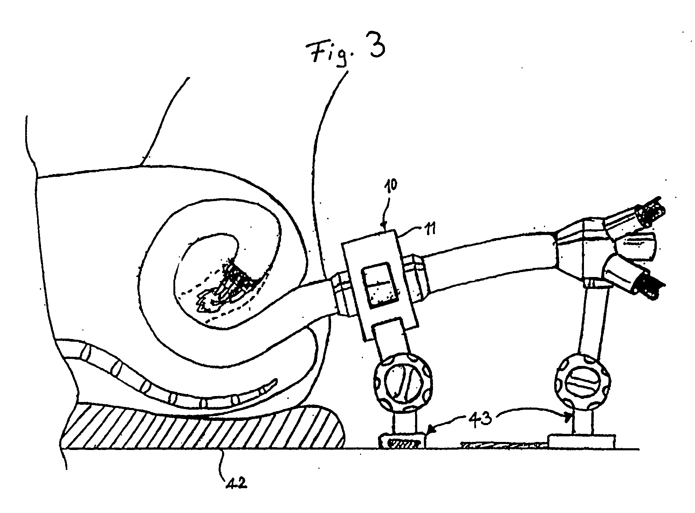

Gemäß Fig. 3 wird zum Einführen des Trokars 1, bzw. des Trokarschafts 1 in den Darm eines Patienten über dessen Anus der Antriebsmechanismus 10, d.h. das Antriebsgehäuse 11 auf einen Sockel 43 montiert, der bezüglich einer Unterlage 42, auf der der Patient zumindest teilweise liegt, - höhenverstellbar und in jede Richtung schwenkbar ist, so daß das distale Ende 3 des Trokarschafts 1 entsprechend dem individuellen Körperbau des Patienten angepaßt werden kann. Anschließend wird das distale Ende 3 des Trokarschafts 1, d.h. ein kurzes Stück in den Anus eingeschoben, solange, bis der Stülpschlauch 2 den Schließmuskel passiert hat. Dabei hat es sich als besonders vorteilhaft erwiesen, die Trokarspitze, d.h. die Vorderkante am distalen Ende des Trokarschafts abzurunden, um so Verletzungen der Darmwand zu vermeiden.

Hierauf wird über den Trokarschaft 1 bzw. den darin geführten Versorgungskanal CO₂-Gas in den Darm eingeleitet, um diesen zu entfalten, worauf die Antriebseinrichtung 10 betätigt wird, um die Reibräder 14 (nicht gezeigt) mit einer vorbestimmbaren oder während des Betriebs auch änderbaren Umdrehungszahl anzutreiben. Entsprechend dieser Umdrehungszahl wird der innere Schlauchabschnitt 2a langsam in Eindringrichtung des Trokars 1 vorgeschoben, wobei er sich im vorderen Umstülpbereich 2d kontinuierlich zu dem vorderen, äußeren Schlauchabschnitt 2b umstülpt und dabei die Darmwandung seitlich auskleiden kann. Gleichzeitig wird die Antriebskraft der Reibräder 14 auch auf den Trokarschaft 1 übertragen, da durch die Anpreßkraft der Reibräder 14 auf den relativ weichen inneren Stülpschlauchabschnitt 2a dieser auf den Trokarschaft 1 angedrückt wird und diesen trotz des dazwischen sich ausbreitenden Schmiermittels durch Reibungskräfte mitnimmt. D. h., durch den Vorschub des inneren Schlauchabschnitts 2a wird der Trokarschaft 1 durch Reibungskräfte zwischen dem Stülpschlauch 2 und dem Schaft 1, gegebenenfalls auch geringfügig durch Druckkräfte in Vorschubrichtung zwischen dem vorderen Umstülpbereich 2d und dem vorderen Klemmstück 7 des Trokarschafts 1 mitgeschoben und somit in den Darm eingeführt.

Um nunmehr die Vorschubgeschwindigkeit des Trokarschafts 1 nicht über die Eindringgeschwindigkeit des Stülpschlauchs 2 hinaus anwachsen zu lassen, wie bereits in der Beschreibungseinleitung ausführlich erläutert wurde, ist ein Rückhalten oder Abbremsen des Trokarschafts 2 gegenüber dem inneren Schlauchabschnitt 2a erforderlich. Dies geschieht - gemäß den Fig. 4-8 vorliegend über das hintere Klemmstück 8, an welches sich der hintere Umstülpbereich 2e des Stülpschlauchs 2 anlegt, der sich zwangsläufig mit der gleichen Geschwindigkeit bewegt, wie der vordere Umstülpbereich 2d und der somit die Vorschubgeschwindigkeit des Trokarschafts 1 bezüglich der Eindringgeschwindigkeit des Stülpschlauchs 2 synchronisiert (d.h. das hintere Klemmstück 8, das fest auf dem Trokarschafts 1 sitzt, hält diesen zurück und leitet dabei die Reaktionskraft in den hinteren Stülpschlauchabschnitt 2c ein). Die Eindringgeschwindigkeit des Stülpschlauchs 2 ist demzufolge gleich groß wie die des Trokarschafts 1.

An dieser Stelle sei darauf hingewiesen, daß während des Synchronisationsvorgangs der Vorschubbewegungen des Stülpschlauchs 2 und des Trokarschafts 1 dieser an den vorderen und hinteren Wülsten wie auch im Bereich der Antriebshülse 5 an dem inneren Stülpschlauchabschnitt 2a gleitet, wobei jedoch die Gleitschwindigkeit des Trokarschafts 1 bezüglich der vorderen und hinteren Wülste, d.h. an den Umstülpbereichen 2d und 2e bzw. gegenüber dem inneren Schlauchabschnitt 2a lediglich der Relativgeschwindigkeit beider Bauteil entspricht, die nur halb so groß ist, wie die Absolutgeschwindigkeit des inneren Stülpschlauchsabschnitts 2a.

Um hierbei die Bremskräfte auf den hinteren Umstülpbereich 2e nicht zu groß werden zu lassen und damit ggf. ein Zusammenstauchen des Stülpschlauchs 2 im hinteren äußeren Schlauchabschnitt 2c durch die aufzubringenden Bremskräfte zu verhindern, ist es erforderlich, die Reibungskräfte zwischen dem Trokarschaft 1 und dem inneren Schlauchabschnitt 2a möglichst gering zu halten.

Wie vorstehend bereits beschrieben wurde, bildet sich in dem Ringspalt 9 zwischen dem Trokar 1 und dem inneren Schlauchabschnitt 2a auch im Bereich der Antriebseinrichtung 10 ein nahezu durchgehender Schmierfilm aus, der gerade noch einen Vortrieb des Trokarschafts 1 im wesentlichen durch die Reibräder 14 ermöglicht, jedoch auch die entstehende Reibung bei der besagten Relativbewegung zwischen den beiden Bauteilen verringert. Darüber hinaus müssen die Umstülpbereiche 2d, 2e und die vorderen und hinteren Klemmstücke 7, 8 aufgrund des geringen Vortriebsanteils auf das vordere Klemmstück 7 bzw. der geringen notwendigen Bremskraft auf das hintere Klemmstück 8 nicht so stark aufeinandergepreßt werden, um beispielsweise eine Dichtwirkung zu erzielen, wie dies bisher im Stand der Technik der Fall war, da dieser Dichtungseffekt bereits durch das Zusammemwirken zwischen den wulstförmigen Umstülpbereichen 2d, 2e und dem Trokarschaft 2 erreicht wird. Im übrigen wird durch die zweite Schmiermittelfördervorrichtung 19 das Schmiermittel in den Hohlraum zwischen dem inneren und äußeren Schlauchabschnitt 2a, 2b, 2c mit einem vorbestimmten Druck gepreßt, so daß hierdurch die Reibungskräfte weiter verringert werden. Hierbei reicht unter Umständen aufgrund geringer Leckage ein anfänglicher Schmiervorgang zur Befüllung des Hohlraums zwischen dem äußeren und inneren Stülpschlauchabschnitt aus, d.h. daß während zumindest einer Behandlungsdauer nicht unbedingt Schmiermittel nachgepreßt werden muß.

Das Zusammenwirken der Reibräder 14 als ein gemeinsamer kontinuierlicher Antrieb für den Stülpschlauch 2 und für den Trokarschaft 1, der vorderen und hinteren Wülste als Dichtungen zusammen mit dem Trokarschaft 1 sowie des durchgehenden Schmierfilms zwischen dem inneren Schlauchabschnitt 2a und dem Schaft 1 erlaubt somit einen äußerst exakten und präzise steuerbaren Vortrieb des Trokarschafts 1.

An dieser Stelle sei darauf hingewiesen, daß an Stelle des hinteren Klemmstücks 8 auch eine zusätzliche externe Synchronisations-Antriebseinrichtung vorgesehen werden kann, die über eine Anzahl von Reibrädern auf den Trokarschaft 1 an dessen hinterem Endabschnitt einwirkt und somit die kontinuierliche Eindringbewegung des Trokars gewährleistet. Des weiteren kann als externer Synchronisations-Antrieb an Stelle der Reibradkonstruktion ein Spindelantrieb für den geregelten kontinuierlichen Vortrieb des Trokarschafts 1 vorgesehen sein.
Um nunmehr das distale Ende 3 des Trokars 1 genau zu plazieren, werden die Bowdenzüge 37 über das Bedienerteil 4 entsprechend betätigt, wodurch sich zumindest das distale Endstück 3 in die gewünschte Richtung krümmt. Hierbei dienen die Kamera-Chips sowie die Kaltlichtquelle mit den zugehörigen Glasfaserkabeln als Überwachungs- und visuelle Untersuchungseinrichtung der zukünftigen Operationsstelle.

Es liegt auf der Hand, daß der flexible Trokar 1 für eine exakte Operation möglichst unbeweglich gehalten werden muß. Eine Möglichkeit der Fixierung besteht darin, über einen weiteren konventionellen Trokar, der im Falle einer Darmoperation durch die Bauchdecke des Patienten eingeführt wird, ein Fixierinstrument an die Außenseite des Darms heranzuführen und über die Darmwand den flexiblen Trokar 1 einzuspannen. Eine andere Möglichkeit wäre, den flexiblen Trokar 1 vorzugsweise unter Röntgen- oder Ultraschallkontrolle auszusteifen. Beispielsweise können die Bowdenzüge 37 gleichzeitig verkürzt werden, wobei sich der Trokar 1 infolge der lockeren Wickelweise der Außenwendel zusammenzieht, bis die Windungen der Außenwendel aneinander anliegen, wodurch der Außenmantel 32 zu einem starren Rohr komprimiert wird. Auch können Versteifungsstreben oder Stangen in freie Funktionskanäle eingezogen werden, um den Außenmantel des Trokar 1 biegesteif zu machen.

Nach der Fixierung des Trokars 1 innerhalb des Darms kann ein Operationsinstrument durch den Innenmantel 33 zur Operationsstelle geführt und unter ständiger Überwachung per Kamera-Chip und/oder per Optik mit der Operation begonnen werden.
Hierfür können beispielsweise die Umrisse der zu entfernenden Geschwulst durch eine Vielzahl von Löchern in die Darmwand per Laser oder einfach durch Messerschnitte markiert werden, wobei diese Löcher durch einen weiteren, über die Bauchdecke eingeführten Trokar aufgrund des durchschimmernden Lichts der Kaltlichtleitungen erkennbar sind. Somit kann die Geschwulst über den äußeren Trokar entfernt werden, ohne daß der Darm aus der Bauchhöhle genommen werden muß. Natürlich ist aber auch eine Operation ausschließlich von Innen, d.h. über den flexiblen Trokar 1 möglich.

Das Zurückziehen des Trokars 1 nach beendeter Operation erfolgt im wesentlichen auf die gleiche Weise wie der Eindringvorgang, wobei in diesem Fall jedoch das vordere Klemmstück 7 die Synchronisation der Bewegungsgeschwindigkeiten zwischen dem Trokarschaft 1 und dem Stülpschlauch 2 übernimmt, während das hintere Klemmstück 8 weitestgehend belastungsfrei ist.

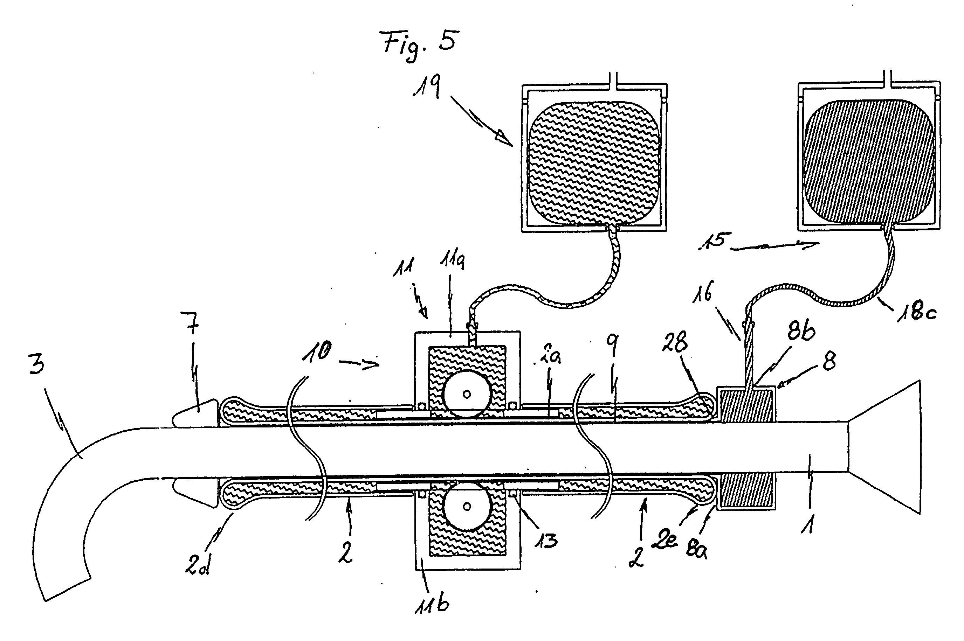

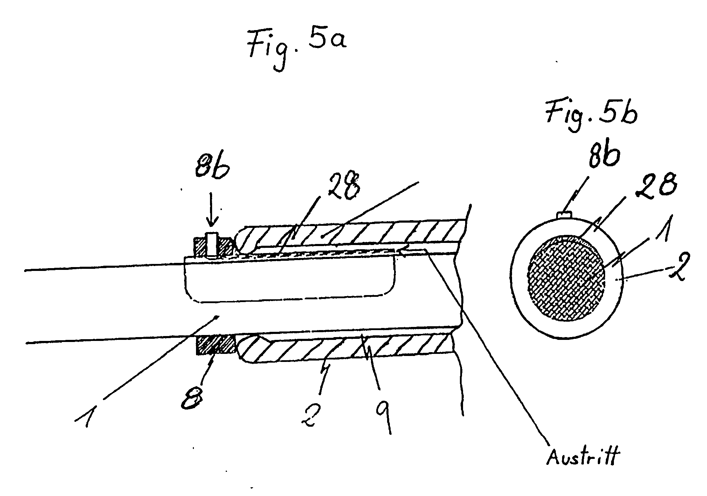

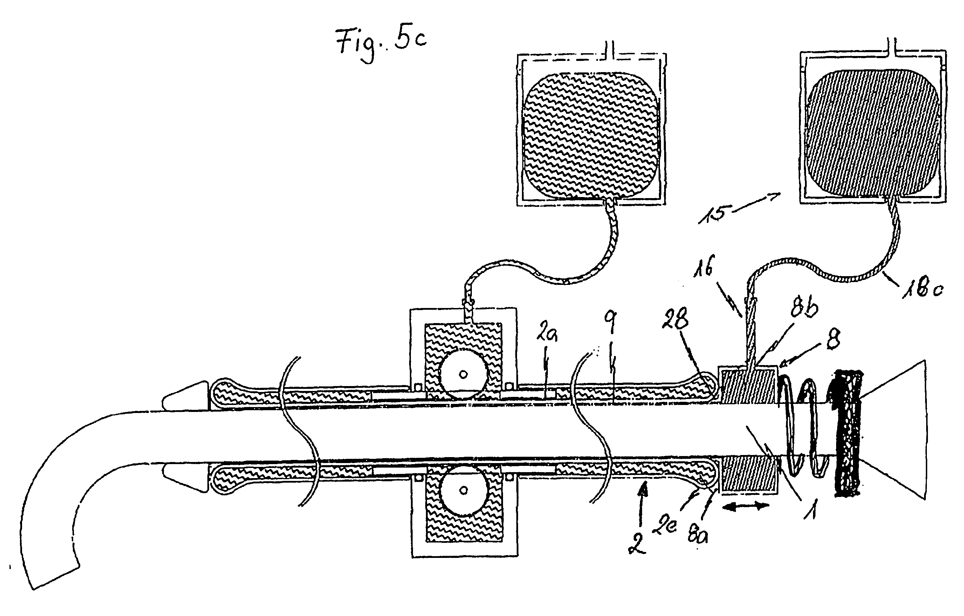

Im nachfolgenden wird ein zweites bevorzugtes Ausführungsbeispiel der Erfindung anhand der Fig. 5, 5a und 5b beschrieben, wobei lediglich auf die zum ersten Ausführungsbeispiel unterschiedlichen Konstruktionsmerkmale eingegangen wird.

Wie in der Fig. 5 gezeigt wird, ist gemäß dem zweiten Ausführungsbeispiel der Erfindung das hintere Klemmstück 8 als eine hohle Manschette ausgeführt, die zusammen mit dem Trokarschaft 1 einen im wesentlichen geschlossenen Hohlraum ausbildet. Vorzugsweise ist die hintere hohle Manschette axial in zwei Halbschalen symmetrisch teilbar, um so besser an den Trokarschaft 1 angelegt werden zu können. Die zum Stülpschlauch 2 gerichtete Seiten- oder Anlagewandung 8a des manschettenförmigen Klemmstücks 8 hat im Bereich des Trokarschafts 1 zumindest einen zum Stülpschlauch 2 vorragenden Schmiermitteleinspritzschuh 28. Dieser Schuh 28 ist insbesondere in Fig. 5b in seinem Querschnitt gezeigt. Demzufolge erstreckt sich der Schmiermitteleinspritzschuh 28 zumindest über einen 1/3-Kreis oder über einen 1/2-Kreis um den Trokarschaft 1. Vorzugsweise sind zwei diametrisch zueinander liegende Schmiermitteleinspritzschuhe 28 vorgesehen, obgleich in Fig. 5b lediglich ein Schuh gezeigt ist. Gemäß der Fig. 5a kann der vorstehend beschriebene Hohlraum der Einfachheit halber auch durch einen Kanal für das Zuführen von Schmiermittel ersetzt sein. Die Schmiermitteleinspritzschuhe 28 liegen ferner zumindest an ihrem äußersten Endabschnitt elastisch auf der äußeren Mantelfläche des Trokarschafts 1 auf und haben jeweils mindestens einen Schmiermittelkanal, wie in der Fig. 5a gezeigt wird, der sich an dem hinteren Ende (Wurzel) des Schmiermitteleinspritzschuhs 28 in den Hohlraum bzw. den Zuführkanal der Manschette und an seinem vorderen freien Ende in den Ringspalt 9 öffnet.

Alternativ zu dem vorstehend beschriebenen Schmiermitteleinspritzschuh 28 können lediglich auch eine Anzahl von Ausnehmungen oder Einkerbungen (nicht näher gezeigt) in der Seitenwand 8a der Manschette 8 vorgesehen sein, die einen Austrittsspalt oder Spalte zwischen dem Trokarschaft 1 und der Anlagewandung 8a bilden.

Des weiteren ist das hintere Klemmstück 8 mit einem Leitungsanschluß 8b versehen, der über die Zufuhrleitung 16 und den Auslaßschlauch 18c mit der ersten Schmiermittelfördervorrichtung 15 fluidverbunden ist. Alle weiteren Teile des Trokar-Stülpschlauch-Systems gemäß der Fig. 5 entsprechen denen des ersten Ausführungsbeispiels.

Während des Betriebs des Trokar-Stülpschlauch-Systems gemäß dem zweiten bevorzugten Ausführungsbeispiel der Erfindung fördert die erste Schmiermittelfördervorrichtung 15 das Schmiermittel in den Hohlraum innerhalb des hinteren manschettenförmigen Klemmstücks 8, der sich entsprechend anfüllt. Bei einem bestimmten Druck innerhalb des Hohlraums strömt das Schmiermittel aus dem Schmiermittelkanal innerhalb des Schmiermitteleinspritzschuhs 28 bzw. aus den Spalten in der Anlagewandung 8a aus und dringt in den Ringspalt 9 zwischen dem Trokarschaft 1 und dem inneren Schlauchabschnitt 2a des Stülpschlauchs 2 zur Schmierung der Relativ-Gleitbewegung beider Bauteile ein. Um eine Leckage am Schmiermitteleinspritzschuh 28 bzw. am Austrittsspalt des hinteren Klemmstücks 8 zu vermeiden, wirkt der hintere Umstülpbereich 2e des Stülpschlauchs 2 als Dichtung, gegen den Trokarschaft 1 und gegen die Anlageseite 8a des hinteren Klemmstücks 8. Auch umschließt der hintere Stülpschlauchbereich der Schmiermitteleinspritzschuh 28 über seine gesamte Länge dichtend ab.

Durch diese Maßnahme erübrigt sich die aufwendige Installation von zusätzlichen Schmiermittelkanälen in dem Zwischraum zwischen dem Außen- 32 und Innenmantel 33 des Trokars 1, wodurch die offene Querschnittsfläch durch die chirugische Instrumente geführt werden, weiter vergrößert ist.

Abschließend sei bezüglich des ersten und zweiten Ausführungsbeispiels der Erfindung darauf hingewiesen, daß insbesondere der Antrieb des Trokars 1 durch Bauteile unterschiedlich zu den Reibrädern 14 erfolgen kann. So könnten beispielsweise für die Vorschubeinrichtung 10 des Stülpschlauchs 2 Zahnräder vorgesehen sein, die auf den inneren Schlauchabschnitt 2a einwirken. All diese Varianten haben jedoch gemeinsam, daß die in Radialrichtung wirkende Anpreßkraft ausreicht, um den inneren Schlauchabschnitt 2a gegen den Trokarschaft 1 für dessen kontinuierlichen Antrieb trotz des Vorhandenseins von Schmiermittel zu drücken, wodurch die exakte Positionierbarkeit des distalen Endes 3 an einer zu untersuchenden und zu operierenden Stelle ermöglicht wird.

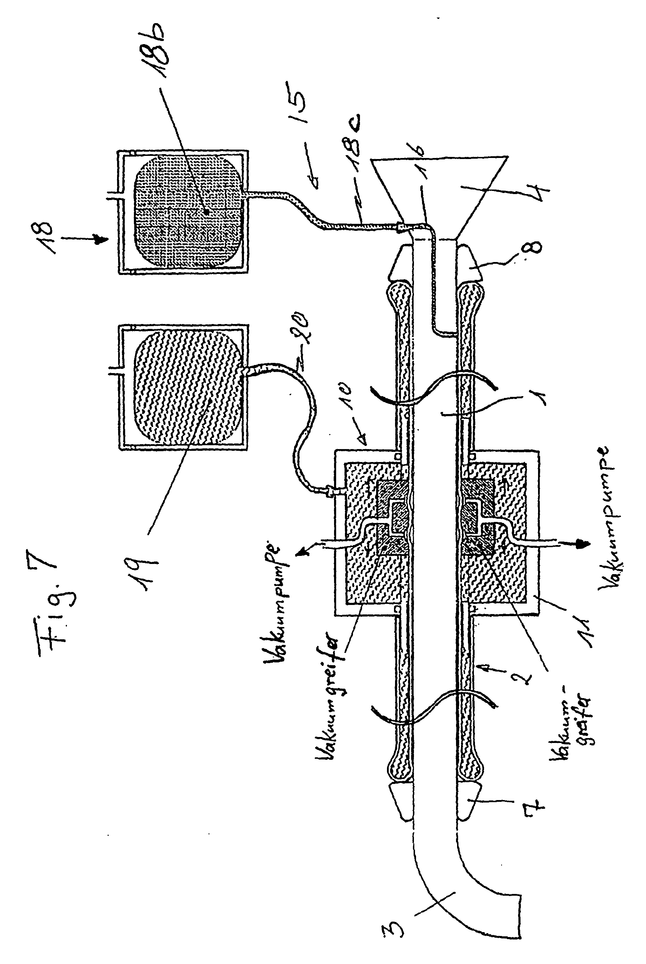

Schließlich wird nachfolgend ein drittes bevorzugtes Ausführungsbeispiel der Erfindung mit Bezug auf die Fig. 7 näher beschrieben, wobei nur auf die Bauteile eingegangen wird, die zu den vorherstehenden Ausführungsbeispielen unterschiedlich sind. Alle weitere Merkmale entsprechen denen des ersten oder zweiten Ausführungsbeispiels.

Gemäß der Fig. 7 besteht die Antriebseinrichtung des dritten Ausführungsbeispiels aus einem Antriebsgehäuse 11, das um die Schlauchführungshülse 5 wie im ersten und zweiten Ausführungsbeispiel gelegt ist und in dem zumindest zwei Vakuumgreifer in Axialrichtung des Trokarschafts 1 alternierend bewegbar angeordnet sind. Die Vakuumgreifer bestehen aus kleinen, durch die Öffnungen in der Antriebshülse 5 durchgreifenden, an dem inneren Stülpschlauchabschnitt 2a dicht anliegenden Klötzchen mit Saugnäpfen, die auf der mit dem Schlauch 2a in Kontakt stehenden Seite der Klötzchen ausgebildet sind und die durch einen von einer Vakuumpumpe erzeugten Unterdruck das Schlauchmaterial im Bereich des inneren Stülpschlauchabschnitts 2a ansaugen und so eine kraft- und formschlüssige Verbindung zum inneren Schlauchabschnitt 2a herstellen. Die Vakuumpumpe (nicht gezeigt) ist hierfür an einen Vakuumanschluß angeschlossen, der im Gehäuse 11 der Antriebseinrichtung 10 ausgebildet und über Kanäle innerhalb der Klötzchen mit den Saugnäpfen verbunden ist. Die Bewegung der Klötzchen, welche durch eine nicht näher gezeigte Bewegungsmechanik bewerkstelligt wird, sowie das Erzeugen des Unterdrucks innerhalb der Saugnäpfe wird derart gesteuert, daß die Klötzchen abwechselnd bzw. bei mehr als zwei Klötzchens in gleichmäßigen Abständen fortlaufend mit entsprechend synchronem Ansaugen und Freigeben des inneren Stülpschlauchabschnitts 2a hin- und herbewegt werden und dabei eine quasi kontinuierliche und gleichmäßige Vorschubbewegung des Stülpschlauchs 2 erzeugen.

Wie in der Fig. 7 gut zu erkennen ist, bewirkt das Vakuumisieren der Saugnäpfe ein geringfügiges Abheben des inneren Stülpschlauchabschnitts 2a vom Trokarschaft. Die Vorschubkraft auf den Trokarschaft 1 erfolgt in diesem Ausführungsbeispiel im wesentlichen wie bei den bisherigen Ausführungen. Die gesamte Anordnung ist dabei wie im ersten und zweiten Ausführungsbeispiel mit einem Schmiermittel gefüllt, das die Schmierung des Stülpschlauchs 2 übernimmt.

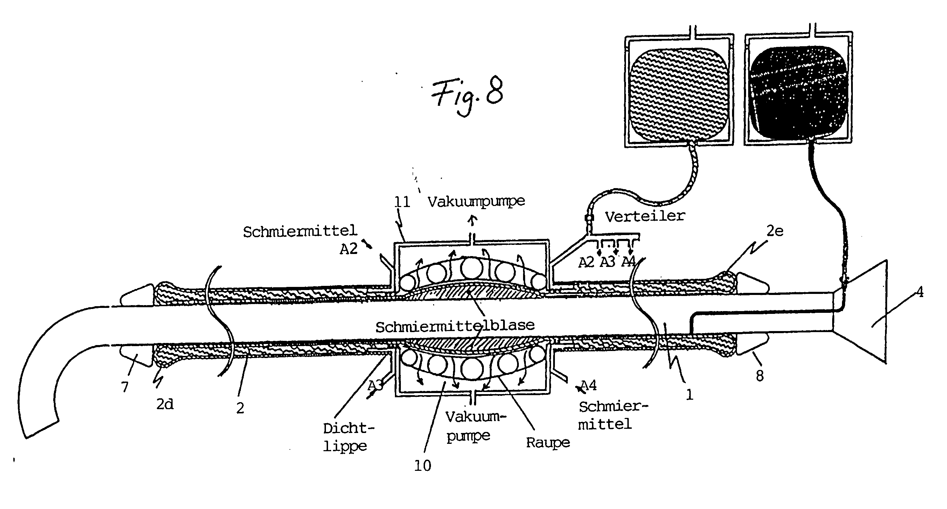

Die Fig. 8 zeigt eine zum dritten Ausführungsbeispiel alternative Ausführungsvariante, bei der durch Erzeugen eines Unterdrucks eine Antriebskraft auf den Stülpschlauch anlegbar ist.

Wie aus der Fig. 8 zu entnehmen ist, bildet der beidseitig umgestülpte Stülpschlauch 2 zusammen mit einem Gehäuse 11 des Antriebsmechanismus ein einstückiges Bauteil ohne die Anordnung einer Antriebshülse. D.h., daß in diesem Ausführungsbeispiel die freien Enden der äußeren vorderen und hinteren Schlauchabschnitte 2b und 2c direkt an den Seitenwänden des Antriebsgehäuses 11 dichtend befestigt sind.

Der Antriebsmechanismus des dritten Ausführungsbeispiels wird dabei durch eine Art Raupenantrieb bestehend aus zumindest einem elastischen Endlosband gebildet, welches über zumindest zwei in Vorschubrichtung voneinander beabstandeter Räder gespannt und angetrieben wird. Die gesamte Antriebseinrichtung ist in dem vorzugsweise in Längsrichtung zweigeteilten, luftdicht verschließbaren Gehäuse untergebracht, welches einen Sauganschluß aufweist. Das Endlosband weist an seiner Oberfläche eine Anzahl von Öffnungen oder eine Perforation auf. Der Sauganschluß ist über eine Schlauchleitung an eine nicht gezeigte Vakuumpumpe angeschlossen.

Das Gehäuse 11 selbst ist auf seiner dem inneren Schlauchabschnitt 2a zugewandten Seite offen, wobei das Endlosband derart geführt ist, daß es die offene Seite des Gehäuses 11 möglichst dicht verschließt. Spalte zwischen den Seitenrändern des Endlosbands und dem Gehäuse 11 können durch geeignete Dichtlippen verschlossen sein, die an den Gehäusewänden befestigt sind und an denen das Endlosband gleitet. Des weiteren ist das Gehäuse 11 bezüglich des daran befestigten Stülpschlauchs 2 derart angeordnet, daß sich die nach außen exponierte Seite des Endlosbands mit einem geringen Spalt oder Abstand oberhalb des inneren Schlauchabschnitts 2a ausrichtet.

Im Betrieb dieser Vorrichtung wird der Innenraum des Gehäuses 11 durch die Vakuumpumpe evakuiert, wobei Luft durch die Perforation in dem Endlosband angesaugt wird. Hierbei wird gleichzeitig der innere Schlauchabschnitt 2a mit angesaugt, der sich dichtend an die Außenseite des Endlosbands anlegt. Durch dieses Abdichten der Perforation erhöht sich der Unterdruck innerhalb des Gehäuses derart, daß sich das Endlosband aufgrund dessen Elastizität nach innen wölbt und dabei den inneren Schlauchabschnitt 2a mitnimmt, wie in der Fig. 8 deutlich gezeigt wird.

Durch diese Maßnahme wird der innere Schlauchabschnitt 2a über die Länge des Endlosbands fest an diesem gehalten und gleichzeitig vom Trokarschaft 1 beabstandet. Der Vortrieb des Trokarschafts 1 erfolgt somit im wesentlichen durch die Anlagekräfte, die bei einer Vorschubbewegung des vorderen äußeren Schlauchabschnitts 2b durch das Endlosband am vorderen Umstülpbereich 2d auf das vordere Klemmstück 7 aufgebracht werden, wobei der innere Schlauchabschnitt 2a insbesondere im Bereich des Antriebseinheit kontaktlos bleibt. Aufgrund der geringen Reibungskräfte ist die notwendige Vorschubkraft auf das vordere Klemmstück 7 relativ niedrig, so daß mit keinem Schuppen (Faltenbildung) im vorderen Abschnitt des Stülpschlauchs 2 zu rechnen ist. Im übrigen wird wie beim ersten und zweiten Ausführungsbeispiel ein Schmiermittel in den Ringspalt 9 gepreßt, wodurch die Reibung insbesondere im Bereich der vorderen und hinteren Dichtungswülste 2d, 2e weiter verringert wird.

Auch das dritte Ausführungsbeispiel wie auch dessen alternative Ausführungsvariante sieht demzufolge wie beim ersten und zweiten Ausführungsbeispiel einen kontinuierlich bzw. quasikontinuierlich arbeitenden mechanisch auf den Stülpschlauch 2 einwirkenden Antriebsmechanismus vor, der eine exakt steuerbare Fortbewegung des Trokars gewährleistet.

Die Erfindung betrifft zusammenfassend einen flexiblen Trokar zur minimal invasiven Operation des Dickdarms über seine gesamte Länge vom Anus aus. Der flexible Trokar besteht aus dem Außenmantel 32 und dem Innenmantel 33. In dem dazwischen befindlichen Raum bzw. Ringspalt verlaufen sämtliche Versorgungs- bzw. Funktionsleitungen. Dazu gehören sowohl die Bowdenzüge 37 zur Verstellung des distalen Endes 3 des Trokars 1, als auch Leitungen für eine Optik, Beleuchtung, Spülung mit Flüssigkeit, Spülung mit Luft, CO₂ o.ä. und Schmierung. Der Innendurchmesser des Innenrohrs 33 ist so bemessen, daß er groß genug ist, mindestens ein oder mehrere Operationswerkzeuge für die minimal invasive Operation hindurch und an die Operationsstelle zu führen. Der Trokar 1 wird mittels eines Stülpschlauchsystems in den Darm eingeführt. Der Antrieb des Schlauchs erfolgt über eine Anzahl von Reibrädern, Saugnäpfen oder Raupen, die auf einen inneren Schlauchabschnitt einwirken, um das System für eine kontinuierliche Bewegung anzutreiben. Zur Abdichtung des Spalts 9 zwischen dem Trokar 1 und dem inneren Schlauchabschnitt bilden vordere und hintere Umstülpbereiche jeweils eine Aufwülstung, die sich dichtend an den Trokar 1 anlegen. Die Zufuhr von Schmiermittel in den Spalt 9 zwischen dem Trokar 1 und innerem Schlauchabschnitt erfolgt über eine im wesentlichen radiale Außenmantelbohrung im Bereich des Stülpschlauchs, die entweder über eine Funktionsleitung an eine externe Zuführleitung angeschlossen ist oder über das hintere Klemmstück mittels eines an die Außenmanteloberfläche angepaßten Schmiermitteleinspritzschuhs.

## Patentansprüche

1. Trokar zum Heranführen von chirurgischen Instrumenten und/oder Diagnoseeinrichtungen,
umfassend
ein flexibles Zugangsrohr (1), das in einen Körperhohlraum einführbar ist und einen flexiblen Außenmantel (32) sowie einen flexiblen Innenmantel (33) hat, die zwischen sich zumindest einen Zwischenraum zur Aufnahme von Versorgungs- und Funktionskanälen ausbilden,
**dadurch gekennzeichnet, dass**
der flexible Trokar eine Einführtiefe bis zumindest zur zweiten Sigmaschleife eines Dickdarms vom Anus aus hat.

2. Trokar nach Anspruch 1,
**dadurch gekennzeichnet, daß**
der Innenmantel (33) coaxial zum Außenmantel (32) angeordnet ist und der Zwischenraum einen Ringspalt ausbildet.

3. Trokar nach Anspruch 2,
**gekennzeichnet durch**
eine Anzahl von axial voneinander beabstandeter Stege in Form von gelochten Ringscheiben, die als Abstandshalter in den Ringspalt eingesetzt sind und die ein Durchführen der Versorgungs- und Funktionskanäle ermöglichen.

4. Trokar nach einem der vorstehenden Ansprüche,
**dadurch gekennzeichnet, daß**
am distalen Ende (3) des flexiblen Zugangsrohres (1) mindestens ein Kamera-Chip (38) oder ein Optiksystem angeordnet ist, das über eine in zumindest einem Versorgungskanal verlegte elektrische oder optische Leitung mit einem Videoprozessor oder einem Sichtgerät verbunden ist.

5. Trokar nach einem der vorhergehenden Ansprüche,
**dadurch gekennzeichnet, daß**
der Zwischenraum mindestens eine Versorungsleitung zur Aufnahme eines Beleuchtungssystems (41) enthält.

6. Trokar nach Anspruch 5,
**dadurch gekennzeichnet, daß**
das Beleuchtungssystem (41) ein Lichtfaserkabel für Kaltlicht ist, das an eine Kaltlichtquelle angeschlossen ist.

7. Trokar nach einem der vorhergehenden Ansprüche,
**dadurch gekennzeichnet, daß**
der Zwischenraum mindestens einen Kanal zur Aufnahme eines Versorgungssystems (39) für das Zu- und/oder Abführen von Luft oder CO₂ enthält.

8. Trokar nach einem der vorhergehenden Ansprüche,
**dadurch gekennzeichnet, daß**
der Zwischenraum mindestens einen Spül- und/oder Saugkanal (40) zur Ver- und Entsorgung des distalen Endes (3) mit Spülflüssigkeit sowie zur Abführung von Körpersekreten, Blut, Körpergewebe usw. enthält.

9. Trokar nach einem der vorhergehenden Ansprüche,
**dadurch gekennzeichnet, daß**
der Außenmantel (32) einen Außendurchmesser von ca. 20-25mm hat.

10. Trokar nach einem der vorhergehenden Ansprüche,
**dadurch gekennzeichnet, daß**
der Außendurchmesser des Außenmantels (32) vorteilhafterweise 22mm beträgt.

11. Trokar nach einem der vorhergehenden Ansprüche,
**dadurch gekennzeichnet, daß**
der Innenmantel (33) eine Einführöffnung mit einem Innendurchmesser von ca. 13-15mm bildet.

12. Trokar nach einem der vorhergehenden Ansprüche,
**dadurch gekennzeichnet, daß**
der Innenmantel (33) vorteilhafterweise eine Innendurchmesser von 14mm hat, wobei zusätzliche rippenförmige Radialvorsprünge an der Innenseite des Trokarschafts in gleichem Umfangsabstand zueinander ausbildbar sind, die den effektiven Innendurchmesser des Innenmantels um ca. 1mm verengen.

13. Trokar nach einem der vorhergehenden Ansprüche,
**dadurch gekennzeichnet, daß**
der Außenmantel (32) eine Wendel aus einem locker gewickelten Flachbandmaterial hat, deren jeweils zwei benachbarte Windungen einen Abstand von ca. 2 bis 3mm aufweisen.

14. Trokar nach Anspruch 13,
**dadurch gekennzeichnet, daß**
als Flachbandmaterial Federstahl oder ein elastischer Kunststoff vorzugsweise mit rechteckigem Profil verwendet ist.

15. Trokar nach einem der Ansprüche 13 oder 14,
**dadurch gekennzeichnet, daß**
die Wendel an ihrer Außenseite mit einer Haut (34) aus einem abriebfesten Kunststoff vorzugsweise aus PVC beschichtet ist, so daß ein Verbundmaterial entsteht.

16. Trokar nach einem der Ansprüche 13 bis 15,
**dadurch gekennzeichnet, daß**
die Wendel an ihrer Innenseite mit einer Haut (36) aus einem Kunststoffmaterial überzogen ist.

17. Trokar nach einem der vorhergehenden Ansprüche,
**dadurch gekennzeichnet, daß**
der Innenmantel (33) aus einer dicht gewickelten Spiralfeder aus einem Federstahldraht oder Kunststoff besteht, wobei jeweils zwei benachbarte Windungen der Spiralfeder eng aneinander liegen.

18. Trokar nach einem der vorhergehenden Ansprüche 1 und 4 bis 17,
**dadurch gekennzeichnet, daß**
der Außenmantel (32) und der Innenmantel (33) im wesentlichen coaxial mit einem vorbestimmten Radialabstand zueinander liegen.

19. Trokar nach einem der vorhergehenden Ansprüche,
**dadurch gekennzeichnet, daß**
die Versorgungs- bzw. Funktionsleitungen symmetrisch oder in gleichen Abständen in Umfangsrichtung in dem Zwischenraum zwischen dem Außenmantel (32) und dem Innenmantel (33) angeordnet sind.

20. Trokar nach einem der vorhergehenden Ansprüche,
**dadurch gekennzeichnet, daß**
der Zwischenraum zwischen dem Innen- und Außenmantel (32, 33) in den Bereichen radial aufgeweitet ist, in denen die Versorgungs- bzw. Funktionsleitungen angeordnet sind, und daß an diesen Stellen radiale Innenvorsprünge als Gleitlager ausgebildet sind.

21. Trokar nach einem der vorstehenden Ansprüche,
**dadurch gekennzeichnet, dass**
in zumindest zwei, vorzugsweise vier Funktionskanälen jeweils ein Bowdenzug (37) verlegt ist, die an ihrem einen Ende am distalen Endabschnitt (3) des Trokar (1) befestigt und an ihrem anderen Ende an einem Bedienerteil (4) zur Betätigung des Trokars (1) angelenkt sind.

22. Stülpschlauchsystem mit einem Trokar (1) mit den Merkmalen gemäß einem der vorstehenden Ansprüche,
**dadurch gekennzeichnet, dass**
der Trokar (1) in einem beidseits gestülpten Schlauch (2) gleitend geführt ist, der durch eine Antriebseinrichtung (10) fortbewegbar ist, die auf einen inneren Schlauchabschnitt (2a) des Stülpschlauchs (2) einwirkt.

23. Stülpschlauchsystem nach Anspruch 22,
**dadurch gekennzeichnet, dass**
die Antriebseinrichtung (10) zumindest ein im Wesentlichen kontinuierlich antreibendes Vorschubmittel (14) hat, das sich radial an den inneren Schlauchabschnitt (2a) anlegt, um diesen in Axialrichtung des Trokars (1) vorwärts und/oder rückwärts zu bewegen.

24. Stülpschlauchsystem nach Anspruch 23,
**dadurch gekennzeichnet, dass**
die Anpresskraft des Vorschubmittels (14) auf den inneren Schlauchabschnitt (2a) so gewählt ist, dass der Trokarschaft (1) zumindest im Bereich des Vorschubmittels (14) in einem Reibungskontakt mit dem inneren Schlauchabschnitt (2a) ist.

25. Stülpschlauchsystem nach Anspruch 24,
**dadurch gekennzeichnet, dass**
die Anpresskraft des Vorschubmittels (14) auf den inneren Stülpschlauchabschnitt (2a) durch eine entsprechende Deformation des inneren Schlauchabschnitts (2a) auf den Trokarschaft (1) übertragbar ist und dieser ein Widerlager für das Vorschubmittel (14) bildet.

26. Stülpschlauchsystem nach einem der vorstehenden Ansprüche 23 und 25,
**dadurch gekennzeichnet, dass**
das Vorschubmittel (14) mindestens ein Reibrad ist, das gegen den inneren Schlauchabschnitt (2a) mit einer vorbestimmten oder variabel einstellbaren Anpresskraft drückt.

27. Stülpschlauchsystem nach Anspruch 26,
**dadurch gekennzeichnet, dass**
zumindest zwei Reibräder (14) vorgesehen sind, die sich bezüglich des Trokarschafts (1) gegenüberliegen.

28. Stülpschlauchsystem nach Anspruch 23,
**dadurch gekennzeichnet, dass**
das Vorschubmittel (14) ein Saugnoppenantrieb ist, bestehend aus zumindest zwei axial zum Trokarschaft (1) hin- und herbewegbaren Klötzchen, deren eine zu dem inneren Stülpschlauchabschnitt (2a) weisende Seitenfläche jeweils mindestens einen Saugnapf aufweist.

29. Stülpschlauchsystem nach Anspruch 28,
**dadurch gekennzeichnet, dass**
die Klötzchen gegenläufig oder nacheinander unter entsprechend synchronem Ansaugen und Loslassen des inneren Stülpschlauchabschnitts (2a) hin- und herbewegbar sind, um hierdurch eine quasi kontinuierliche Vortriebsbewegung des Stülpschlauchs (2) zu erzeugen.

30. Stülpschlauchsystem nach einem der Ansprüche 28 oder 29,
**dadurch gekennzeichnet, dass**
die Antriebskraft der Klötzchen auf den inneren Stülpschlauchabschnitt (2a) über die vorderen oder hinteren Stülpbereiche (2d, 2e) auf ein vorderes oder hinteres Klemmstück (7, 8) übertragbar ist, die fest am Trokarschaft (1) fixiert sind und somit eine entsprechende Vorschubbewegung des Schafts (1) synchron zur Vorschubbewegung des Stülpschlauchs (2) bewirken.

31. Stülpschlauchsystem nach Anspruch 23,
**dadurch gekennzeichnet, dass**
das Vorschubmittel (14) ein Raupenantrieb bestehend aus einem Endlosband ist.

32. Stülpschlauchsystem nach Anspruch 31,
**dadurch gekennzeichnet, dass**
das Endlosband in einem Antriebsgehäuse (11) gelagert ist, das zumindest in einem durch das Endlosband im wesentlichen luftdicht abgeschlossenen Gehäuseteil evakuierbar ist.

33. Stülpschlauchsystem nach Anspruch 31,
**dadurch gekennzeichnet, dass**
das Endlosband eine zum inneren Stülpschlauchabschnitt (2a) weisende Gehäuseseite bildet und den Gehäuseinnenraum nahezu luftdicht verschließt.

34. Stülpschlauchsystem nach einem der Ansprüche 31 bis 33,
**dadurch gekennzeichnet, dass**
das Endlosband gelocht oder perforiert ist.

35. Stülpschlauchsystem nach Anspruch 34,
**dadurch gekennzeichnet, dass**
bei einem Evakuieren des Antriebsgehäuses (11) der innere Stülpschlauchabschnitt (2a) über die Perforation an das Endlosband ansaugbar ist, wobei sich das Endlosband zusammen mit dem inneren Schlauchabschnitt (2a) geringfügig in das Antriebsgehäuse wölbt.

36. Stülpschlauchsystem nach einem der Ansprüche 22 bis 35,
**dadurch gekennzeichnet, dass**
der Stülpschlauch (2) an seinem vorderen Stülpbereich (2d) gegen ein vorderes Klemmstück (7) des Trokarschafts (1) anliegt, um zumindest eine Teilvorschubkraft an den Trokarschaft (1) anzulegen.

37. Stülpschlauchsystem nach einem der vorstehenden Ansprüche 22 bis 36,
**dadurch gekennzeichnet, dass**
die Antriebseinrichtung (10) ferner eine Vorrichtung zur Synchronisation der Trokarschaftbewegung mit der Stülpschlauchbewegung umfasst.

38. Stülpschlauchsystem nach Anspruch 37,
**dadurch gekennzeichnet, dass**
die Synchronisationsvorrichtung ein am Trokarschaft (1) axial fixiertes vorderes und hinteres Klemmstück (7, 8) ist, an dem gleitfähig der hintere Stülpbereich (2e) des Stülpschlauchs (2) bei einer Vorwärtsbewegung und der vordere Stülpbereich (2d) bei einer Rückwärtsbewegung anliegt, so dass der Stülpschlauch (2) über das jeweilige Klemmstück (7 oder 8) eine Bremskraft entgegen der Vorschubkraft auf den Trokarschaft (1) aufbringt.

39. Stülpschlauchsystem nach Anspruch 37,
**dadurch gekennzeichnet, dass**
die Synchronisationsvorrichtung ein auf den hinteren Endabschnitt des Trockarschafts (1) einwirkender Rollen- oder Spindelantrieb ist, der mit dem Stülpschlauchantrieb (10) derart synchronisiert ist, dass die Vorschubgeschwindigkeit des Trockarschafts (1) die Hälfte der Vorschubgeschwindigkeit des inneren Schlauchabschnitts (2a) beträgt.

40. Stülpschlauchsystem gemäß Anspruch 22,
**dadurch gekennzeichnet, dass**
der Stülpschlauch (2) eine Schlauchführungshülse (5) aus einem steifen Material hat, die über den inneren Schlauchabschnitt (2a) unter Ausbildung eines Zwischenspalts gezogen ist und an deren beiden axialen Endabschnitten die freien Enden äußerer Schlauchabschnitte (2b, 2c) fixiert sind, die durch Umstülpen und Zurückführen des inneren Schlauchabschnitts (2a) an zwei axial beabstandeten Umstülpbereichen (2d, 2e gebildet werden.

41. Stülpschlauchsystem nach Anspruch 40,
**dadurch gekennzeichnet, dass**
die Hülse (5) in ihrem Mittenabschnitt eine Anzahl von Öffnungen hat, die vorzugsweise in Form von Längsschlitzen (6) ausgebildet und in gleichem Winkelabstand zueinander angeordnet sind.

42. Stülpschlauchsystem nach Anspruch 41,
**dadurch gekennzeichnet, dass**
der Stülpschlauch (2) über den inneren Schlauchabschnitt (2a), die äußeren Schlauchabschnitte (2b, 2c) sowie die Hülse (5) einen gegenüber der Umgebung abgedichteten Hohlraum ausbildet, der lediglich über die Öffnungen (6) in der Hülse (5) einen Zugang hat.

43. Stülpschlauchsystem nach einem der Ansprüche 40 bis 42,
**dadurch gekennzeichnet, dass**
die Hülse (5) an ihrer Innenseite eine Anzahl von im wesentlichen in Axialrichtung verlaufender Nuten (5a) hat, die sich an den Stirnflächen der Hülse (5) öffnen.

44. Stülpschlauchsystem gemäß Anspruch 22,
**gekennzeichnet durch**
eine Schmiervorrichtung (15) für das Einpressen von Schmiermittel in einen Ringspalt (9) zwischen dem Trokar bzw. Trokarschafts (1) und dem inneren Stülpschlauchabschnitt (2a), wobei zumindest eine im wesentlichen radial verlaufende Öffnung (17) oder Perforation in dem Außenmantel (32) des Trokars (1) vorgesehen ist, die sich in den Ringspalt (9) öffnet und an einer Schmiermittelfördervorrichtung (15) angeschlossen ist.

45. Stülpschlauchsystem gemäß Anspruch 44,
**dadurch gekennzeichnet, dass**
zwischen dem Außenmantel (32) und dem Innenmantel (33) des Trokars (1) mindestens eine Schmierstoffleitung als ein weiterer Funktionskanal vorgesehen ist, die in Axialrichtung in bestimmten Abständen radiale Durchbrüche durch den Außenmantel (33) aufweist, um die Schmiermittelversorgung von der Außenwand des Trokars (1) und der Innenseite des Stülpschlauches über die gesamte Länge des Trokars (1) zu gewährleisten und die an eine Schmiermittelfördervorrichtung anschließbar ist.

## Claims

1. A trocar for introducing surgical instruments and/or diagnostic devices, comprising a flexible access tube (1), which can be inserted into a body cavity and has a flexible external jacket (32) and a flexible internal jacket (33) which between them form at least one space for supply and function channels, **characterized in that**
the flexible trocar can be inserted at least to the second sigma loop of a colon from the anus.

2. A trocar as claimed in claim 1, **characterized in that** the internal jacket (33) is coaxial to the external jacket (32) and the space forms an annular gap.

3. A trocar as claimed in claim 2, **characterized by** a number of axially separated links in the form of perforated washers, which are inserted as spreaders into the annular gap and enable the insertion of the supply and function channels.

4. A trocar as claimed in one of the preceding claims, **characterized in that** at least one camera chip (38) or an optical system is located at the distal end (3) of the flexible access tube (1), and is connected to a videoprocessor or a visual display unit by an electrical or optical line placed in at least one supply channel.

5. A trocar as claimed in one of the preceding claims, **characterized in that** the space contains at least one supply line for a lighting system (41).

6. A trocar as claimed in claim 5, **characterized in that** the lighting system (41) is an optical fiber cable for cold light, which is connected to a cold-light source.

7. A trocar as claimed in one of the preceding claims, **characterized in that** the space contains at least one channel for a supply system (39) for introducing and removing air or CO₂.

8. A trocar as claimed in one of the preceding claims, **characterized in that** the space contains at least one flushing and/or suction channel (40) for supplying and removing a flushing liquid to the distal end (3), and to remove body secretions, blood, body tissue etc.

9. A trocar as claimed in one of the preceding claims, **characterized in that** the external jacket (32) has an outside diameter of about 20 - 25 mm.

10. A trocar as claimed in one of the preceding claims, **characterized in that** the outside diameter of the external jacket (32) is advantageously 22 mm.

11. A trocar as claimed in one of the preceding claims, **characterized in that** the internal jacket (33) forms an insertion opening with an inside diameter of about 13 - 15 mm.

12. A trocar as claimed in one of the preceding claims, **characterized in that** the internal jacket (33) advantageously has an inside diameter of 14 mm, where additional rib-shaped radial protrusions can be formed inside the trocar shaft at an equal circumferential distance from each other, which narrow the inside diameter of the internal jacket by about 1 mm.

13. A trocar as claimed in one of the preceding claims, **characterized in that** the external jacket (32) has a helix made of a loosely wound flat strip material, where any two adjacent windings are separated by about 2 to 3 mm.

14. A trocar as claimed in claim 13, **characterized in that** spring steel or a flexible plastic material is used as the flat strip material, preferably with a rectangular profile.

15. A trocar as claimed in one claims 13 or 14, **characterized in that** the outside of the helix is coated with a film (34) of an abrasion-resistant plastic material, preferably PVC, thereby creating a compound material.

16. A trocar as claimed in one claims 13 to 15, **characterized in that** the inside of the helix is covered with a film (36) of a plastic material.

17. A trocar as claimed in one of the preceding claims, **characterized in that** the internal jacket (33) consists of a tightly wound spiral spring made of a steel spring wire or a plastic material, where any two adjacent windings of the spiral spring lie close to each other.

18. A trocar as claimed in one of the preceding claims 1 and 4 to 17, **characterized in that** the external jacket (32) and the internal jacket (33) are essentially coaxial at a predetermined radial distance from each other.

19. A trocar as claimed in one of the preceding claims, **characterized in that** the supply and function channels are located symmetrically or at equal distances in the circumferential direction in the space between the external jacket (32) and the internal jacket (33).

20. A trocar as claimed in one of the preceding claims, **characterized in that** the space between the internal and external jacket (32, 33) is radially expanded in the areas where the supply and function channels are located, and that inner radial protrusions form slide bearings in these areas.

21. A trocar as claimed in one of the preceding claims, **characterized in that** at least two, preferably four function channels contain one Bowden cable (37) each, where one end is attached to the distal end-section (3) of the trocar (1), and the other end is coupled to an actuation device (4) for operating the trocar (1).

22. An invertible hose system with a trocar (1) including the features claimed in one of the preceding claims, **characterized in that** the trocar (1) slides in a hose (2) inverted at both ends which advance through a driving device (10) and acts on an inner hose section (2a) of the invertible hose (2).

23. An invertible hose system as claimed in claim 22, **characterized in that** the driving device (10) has at least one advance mechanism (14) which essentially drives continuously and abuts radially against the inner hose section (2a) to move it forward and/or backward in the axial direction of the trocar (1).

24. An invertible hose system as claimed in claim 23, **characterized in that** the pressure force exerted by the advance mechanism (14) on the inner hose section (2a) is designed so that the trocar shaft (1) makes frictional contacts with the inner hose section (2a), at least in the area of the advance mechanism (14).

25. An invertible hose system as claimed in claim 24, **characterized in that** the pressure force exerted by the advance mechanism (14) on the inner hose section (2a) can be transmitted by a corresponding deformation of the inner hose section (2a) to the trocar shaft (1), and the latter forms an abutment for the advance mechanism (14).

26. An invertible hose system as claimed in one of the preceding claims 23 to 25, **characterized in that** the advance mechanism (14) is at least one friction wheel which exerts a predetermined or variable pressure force against the inner hose section (2a).

27. An invertible hose system as claimed in claim 26, **characterized in that** at least two friction wheels (14) are provided, which oppose each other with respect to the trocar shaft (1).

28. An invertible hose system as claimed in claim 23, **characterized in that** the advance mechanism (14) is a suction cup drive, consisting of at least two small blocks that move back and forth axially with respect to the trocar shaft (1), where each of its lateral surfaces facing the inner invertible hose section (2a) has at least one suction cup.

29. An invertible hose system as claimed in claim 28, **characterized in that** the small blocks can be reciprocated in an opposite or successive manner, subject to a corresponding synchronous pull or release of the inner invertible hose section (2a), thereby producing a quasi continuous advance of the invertible hose (2).

30. An invertible hose system as claimed in one of claims 28 or 29, **characterized in that** the driving force of the small blocks on the inner invertible hose section (2a) via the front or rear of invertible areas (2d, 2e) can be transmitted to a front or rear clamping part (7, 8) which is securely affixed to the trocar shaft (1), thereby causing a corresponding advance of the shaft (1) which is synchronous with the advance of the invertible hose (2).

31. An invertible hose system as claimed in claim 23, **characterized in that** the advance mechanism (14) is a caterpillar drive consisting of an endless band.

32. An invertible hose system as claimed in claim 31, **characterized in that** the endless band is located in a drive housing (11), part of which is essentially sealed air-tight by the endless band and can be evacuated.

33. An invertible hose system as claimed in claim 31, **characterized in that** the endless band forms a side of the housing facing the inner invertible hose section (2a) which seals the internal housing space nearly air-tight.

34. An invertible hose system as claimed in one of claims 31 to 33, **characterized in that** the endless band is perforated.

35. An invertible hose system as claimed in claim 34, **characterized in that** when evacuating the drive housing (11), the inner invertible hose section (2a) can be sucked through the perforation against the endless band, where the latter and the inner hose section (2a) curve slightly into the drive housing.

36. An invertible hose system as claimed in one of claims 32 to 35, **characterized in that** the invertible front area (2d) of the invertible hose (2) abuts against a front clamping part (7) of the trocar shaft (1), imparting at least a partial advance to the trocar shaft (1).

37. An invertible hose system as claimed in one of the preceding claims 22 to 36, **characterized in that** the driving device (10) also comprises a device for synchronizing the trocar shaft movement with the invertible hose movement.

38. An invertible hose system as claimed in claim 37, **characterized in that** the synchronization device is a front and rear clamping part (7, 8) axially affixed to the trocar shaft (1), against which the rear invertible area (2e) of invertible hose (2) slides and abuts during a forward movement, and the front invertible area (2d) during a rearward movement, causing the invertible hose (2) to exert a braking force on the trocar shaft (1) via the respective clamping part (7 or 8).

39. An invertible hose system as claimed in claim 37, **characterized in that** the synchronization device is a roller or spindle drive which acts on the rear end-section of the trocar shaft (1), and is synchronized with the invertible hose drive (10) so that the advance speed of the trocar shaft (1) is half the advance speed of the inner hose section (2a).

40. An invertible hose system as claimed in claim 22, **characterized in that** the invertible hose (2) has a guidance bushing (5) made of a stiff material, which is placed over the inner hose section (2a) and forms an internal gap, where the free ends of outer hose sections (2b, 2c) are affixed to the bushing's two axial end-sections, forming two axially separated inverted areas (2d, 2e) by inverting and returning the inner hose section (2a).

41. An invertible hose system as claimed in claim 40, **characterized in that** the central section of the bushing (5) has a number of openings which are preferably lengthwise slits (6) placed at equal angular distances from each other.

42. An invertible hose system as claimed in claim 41, **characterized in that,** with the inner hose section (2a), the outer hose sections (2b, 2c) as well as the bushing, the invertible hose (2) forms a hollow space that is sealed against the outside and can only be accessed through the openings (6) in the bushing (5).

43. An invertible hose system as claimed in one of claims 40 to 42, **characterized in that** the inside of the bushing (5) has a number of axially running grooves (5a), which open to the front faces of the bushing (5).

44. An invertible hose system as claimed in claim 22, **characterized by** a lubrication device (15) for pressing lubricant into an annular gap (9) between the trocar or the trocar shaft (1) and the inner invertible hose section (2a), having at least one essentially radially extending opening (17) or perforation in the external jacket (32) of the trocar (1), which opens to the annular gap (9) and is connected to a lubricant conveying device (15).

45. An invertible hose system as claimed in claim 44, **characterized in that** as a further function channel between the external jacket (32) and the internal jacket (33) of the trocar (1), at least one lubricant line is provided with radial break-throughs in the external jacket (33) at predetermined distances in the axial direction, to ensure the supply of lubricant from the external wall of the trocar (1) and the inside of the invertible hose along the entire length of the trocar (1), and can be connected to a lubricant conveying device.

## Revendications

1. Trocart pour l'amenée d'instruments chirurgicaux et/ou de dispositifs de diagnostic,
comprenant :
un tube d'accès (1) flexible, susceptible d'être introduit dans un espace creux corporel et comprenant une enveloppe extérieure (32) flexible, ainsi qu'une enveloppe intérieure (33) flexible, constituant entre eux au moins un espace intermédiaire pour recevoir des canaux d'alimentation et fonctionnels,
**caractérisé en ce que**
le trocart flexible présente une profondeur d'introduction allant jusqu'au moins la deuxième boucle sigma d'un gros intestin, à partir de l'anus.

2. Trocart selon la revendication 1,
**caractérisé en ce que**
l'enveloppe intérieure (33) est disposée coaxialement à l'enveloppe extérieure (32) et l'espace intermédiaire forme un intervalle annulaire.

3. Trocart selon la revendication 2,
**caractérisé par**
une pluralité de nervures espacées axialement les unes des autres, ayant la forme de disques annulaires perforés, insérés, en tant que support d'espacement, dans l'intervalle annulaire et permettant un passage des canaux d'alimentation et fonctionnels.

4. Trocart selon l'une des revendications précédentes,
**caractérisé en ce que**
à l'extrémité distale (3) du tube d'accès (1) flexible est disposé au moins une puce électronique formant caméra (38), ou un système optique, relié(e) à un processeur vidéo ou un appareil de visualisation, par l'intermédiaire d'une ligne électrique ou optique, posée dans au moins un canal d'alimentation.

5. Trocart selon l'une des revendications précédentes,
**caractérisé en ce que**
l'espace intermédiaire contient au moins une conduite d'alimentation, pour recevoir un système d'éclairage (41).

6. Trocart selon la revendication 5,
**caractérisé en ce que**
le système d'éclairage (41) est un câble à fibre optique pour lumière froide, raccordé à une source de lumière froide.

7. Trocart selon l'une des revendications précédentes,
**caractérisé en ce que**
l'espace intermédiaire contient au moins un canal pour recevoir un système d'alimentation (39) pour l'amenée et/ou l'évacuation d'air ou de CO₂.

8. Trocart selon l'une des revendications précédentes,
**caractérisé en ce que**
l'espace intermédiaire contient au moins un canal de rinçage et/ou d'aspiration (40), pour l'amenée et l'évacuation de liquide de rinçage à l'extrémité distale (3), ainsi que pour l'évacuation de sécrétions corporelles, sang, tissu corporel, etc.

9. Trocart selon l'une des revendications précédentes,
**caractérisé en ce que**
l'enveloppe extérieure (32) présente un diamètre extérieur d'à peu près 20 à 25 mm.

10. Trocart selon l'une des revendications précédentes,
**caractérisé en ce que**
le diamètre extérieur de l'enveloppe extérieure (32) est avantageusement de 22 mm.

11. Trocart selon l'une des revendications précédentes,
**caractérisé en ce que**
l'enveloppe intérieure (33) forme une ouverture d'insertion d'un diamètre intérieur d'à peu près 13 à 15 mm.

12. Trocart selon l'une des revendications précédentes,
**caractérisé en ce que**
l'enveloppe intérieure (33) présente avantageusement un diamètre intérieure de 14 mm, sachant que des saillies radiales en forme d'ailettes supplémentaires sont réalisables, selon le même espacement périphérique les unes par rapport aux autres, sur la face intérieure de la tige de trocart, ayant comme effet de rétrécir d'à peu près 1 mm le diamètre intérieur effectif de l'enveloppe intérieure.

13. Trocart selon l'une des revendications précédentes,
**caractérisé en ce que**
l'enveloppe extérieure (32) comprend un enroulement formé d'un matériau en ruban plat enroulé de façon lâche, dont chaque fois deux enroulements voisins présentent un espacement d'à peu près 2 à 3 mm.

14. Trocart selon la revendication 13,
**caractérisé en ce que**
l'on utilise comme matériau en ruban plat de l'acier à ressort ou une matière synthétique élastique, de préférence ayant un profil rectangulaire.

15. Trocart selon l'une des revendications 13 ou 14,
**caractérisé en ce que,**
sur sa face extérieure, l'enroulement est revêtu d'une peau (34) en une matière synthétique résistant à l'abrasion, de préférence formée de PVC, de sorte qu'est créé un matériau composite.

16. Trocart selon l'une des revendications 13 à 15,
**caractérisé en ce que**
l'enroulement est revêtu sur sa face intérieure d'une peau (36) en un matériau synthétique.

17. Trocart selon l'une des revendications précédentes,
**caractérisé en ce que**
l'enveloppe intérieure (33) est composée d'un ressort spiral enroulé de façon serrée, composé d'un fil d'acier à ressort ou de matière synthétique, deux enroulement voisins du ressort spiral étant chaque fois en appui étroit l'un sur l'autre.

18. Trocart selon l'une des revendications précédentes 1 et 4 à 17,
**caractérisé en ce que**
l'enveloppe extérieure (32) et l'enveloppe intérieure (33) sont placées sensiblement coaxialement l'une par rapport à l'autre, avec un espacement radial prédéterminé.

19. Trocart selon l'une des revendications précédentes,
**caractérisé en ce que**
les conduites d'alimentation ou fonctionnelles sont disposées symétriquement ou selon des espacements identiques en direction périphérique, dans l'espace intermédiaire existant entre l'enveloppe extérieure (32) et l'enveloppe intérieure (33).

20. Trocart selon l'une des revendications précédentes,
**caractérisé en ce que**
l'espace intermédiaire, existant entre l'enveloppe extérieure (32) et l'enveloppe intérieure (33), est élargi radialement dans les zones dans lesquelles les conduites d'alimentation ou fonctionnelles sont disposées, et **en ce que**, à cet emplacement, sont réalisées des saillies intérieures radiales, faisant office de paliers à glissement.

21. Trocart selon l'une des revendications précédentes,
**caractérisé en ce que,**
en au moins deux, de préférence quatre canaux fonctionnels est chaque fois posé un câble de Bowden (37), ceux-ci étant fixés, à une de leurs extrémités, sur le tronçon d'extrémité distale (3) du trocart (1) et articulés, à l'autre extrémité, à une partie de manoeuvre (4), pour l'actionnement du trocart (1).

22. Système à tuyau à retournement, avec un trocart (1) présentant les caractéristiques selon l'une des revendications précédentes,
**caractérisé en ce que**
le trocart (1) est guidé avec glissement dans un tuyau (2) à retournement des deux côtés, susceptible d'être avancé au moyen d'un dispositif d'entraînement (10), agissant sur un tronçon de tuyau intérieur (2a) du tuyau à retournement (2) .

23. Système à tuyau à retournement selon la revendication 22,
**caractérisé en ce que**
le dispositif d'entraînement (10) comprend au moins un moyen d'avancement (14), entraînant de façon sensiblement continue, s'appliquant radialement sur le tronçon de tuyau intérieur (2a), pour déplacer celui-ci en avant et/ou en arrière dans la direction axiale du trocart (1).

24. Système à tuyau à retournement selon la revendication 23,
**caractérisé en ce que**
la force de pressage, du moyen d'avancement (14) sur le tronçon de tuyau intérieur (2a), est choisie de manière que la tige de trocart (1), au moins dans la zone du moyen d'avancement (14), soit en contact de friction avec le tronçon de tuyau intérieur (2a).

25. Système à tuyau à retournement selon la revendication 24,
**caractérisé en ce que**
la force de pressage, du moyen d'avancement (14) sur le tronçon de tuyau à retournement intérieur (2a), est transmissible à la tige de trocart (1), au moyen d'une déformation correspondante du tronçon de tuyau intérieur (2a), et celle-ci forme un contre-appui pour le moyen d'avancement (14).

26. Système à tuyau à retournement selon l'une des revendications 23 et 25 précédentes,
**caractérisé en ce que**
le moyen d'avancement (14) est au moins une roue à friction, pressant contre le tronçon de tuyau intérieur (2a) avec une force de pressage prédéterminée ou réglable à une valeur variable.

27. Système à tuyau à retournement selon la revendication 26,
**caractérisé en ce qu'**
au moins deux roues à friction (14) sont prévues, placées à l'opposé par rapport à la tige de trocart (1).

28. Système à tuyau à retournement selon la revendication 23,
**caractérisé en ce que**
le moyen d'avancement (14) est un entraînement à ventouses, composé d'au moins deux blocs, déplaçables dans un sens et dans l'autre axialement par rapport à la tige de trocart (1), dont une face latérale, tournée vers le tronçon de tuyau à retournement intérieur (2a), présente chaque fois au moins une ventouse.

29. Système à tuyau à retournement selon la revendication 28,
**caractérisé en ce que**
les blocs sont déplaçables dans un sens et dans l'autre, en sens inverse ou l'un à la suite de l'autre, avec une aspiration et un relâchement synchrone correspondant du tronçon de tuyau à retournement intérieur (2a), de manière à produire de ce fait un déplacement d'avancement quasi continu du tuyau à retournement (2).

30. Système à tuyau à retournement selon l'une des revendications 28 ou 29,
**caractérisé en ce que**
la force d'entraînement des blocs, sur le tronçon de tuyau à retournement intérieur (2a), est transmissible, par l'intermédiaire des zones à retournement avant ou arrière (2d, 2e), à une pièce de serrage avant ou arrière (7, 8), les pièces de serrage étant fixées rigidement sur la tige de trocart (1) et provoquant ainsi un déplacement d'avancement correspondant de la tige (1), de manière synchrone par rapport au déplacement du tuyau à retournement (2).

31. Système à tuyau à retournement selon la revendication 23,
**caractérisé en ce que**
le moyen d'avancement (14) est un entraînement à chenille, composée d'une bande sans fin.

32. Système à tuyau à retournement selon la revendication 31,
**caractérisé en ce que**
la bande sans fin est montée dans un boîtier d'entraînement (11), susceptible d'être placé sous vide, au moins dans une partie de boîtier, fermée de façon pratiquement étanche à l'air au moyen de la bande sans fin.

33. Système à tuyau à retournement selon la revendication 31,
**caractérisé en ce que**
la bande sans fin forme une face de boîtier tournée vers le tronçon de tuyau à retournement intérieur (2a) et ferme de façon pratiquement étanche à l'air l'espace intérieur du boîtier.

34. Système à tuyau à retournement selon l'une des revendications 31 à 33,
**caractérisé en ce que**
la bande sans fin est percée ou perforée.

35. Système à tuyau à retournement selon la revendication 34,
**caractérisé en ce que**, lors de la mise sous vide du boîtier d'entraînement (11), le tronçon de tuyau à retournement intérieur (2a) est susceptible d'être plaqué sur la bande sans fin, par aspiration, sur la perforation, la bande sans fin, conjointement avec le tronçon de tuyau à retournement intérieur (2a), s'incurvant légèrement dans le boîtier d'entraînement.

36. Système à tuyau à retournement selon l'une des revendications 22 à 35,
**caractérisé en ce que**
le tuyau à retournement (2), sur sa zone à retournement avant (2d), appuie contre une pièce de serrage avant (7) de la tige de trocart (1), pour appliquer au moins une force partielle d'avancement à la tige de trocart (1).

37. Système à tuyau à retournement selon l'une des revendications 22 à 36,
**caractérisé en ce que**
le dispositif d'entraînement (10) comprend en outre un dispositif pour la synchronisation du déplacement de la tige de trocart avec le déplacement du tuyau à retournement.

38. Système à tuyau à retournement selon la revendication 37,
**caractérisé en ce que**
le dispositif de synchronisation est une pièce de serrage avant et arrière (7, 8), fixée axialement à la tige de trocart (1), pièce de serrage sur laquelle appuie, avec possibilité de glissement, la zone à retournement arrière (2e) du tuyau à retournement (2), lors d'un déplacement d'avancement, et la zone à retournement avant (2d), lors d'un déplacement de recul, de manière que le tuyau à retournement (2) applique sur la tige de trocart (1), par l'intermédiaire de la pièce de serrage (7 ou 8) respective, une force de freinage à l'encontre de la force d'avancement.

39. Système à tuyau à retournement selon la revendication 37,
**caractérisé en ce que**
le dispositif de synchronisation est un entraînement à galet ou à broche, agissant sur le tronçon d'extrémité arrière de la tige de trocart (1), entraînement synchronisé avec l'entraînement de tuyau à retournement (10), de manière que la vitesse d'avancement de la tige de trocart (1) soit de la moitié de la vitesse d'avancement du tronçon de tuyau intérieur (2a).

40. Système à tuyau à retournement selon la revendication 22,
**caractérisé en ce que**
le tuyau à retournement (2) comprend une douille de guidage de tuyau (5) composée d'un matériau rigide, tirée sur le tronçon de tuyau intérieur (2a) en formant un intervalle intermédiaire et sur les deux tronçons d'extrémité axiaux de laquelle sont fixées les extrémités libres de tronçons de tuyau extérieurs (2b, 2c), formées par retournement et rétraction du tronçon de tuyau intérieur (2a) en deux zones de retournement (2d, 2e) espacées axialement.

41. Système à tuyau à retournement selon la revendication 40,
**caractérisé en ce que**
la douille (5) présente sur son tronçon central une pluralité d'ouvertures, ayant de préférence la forme de fentes allongées (6) et disposées selon le même espacement angulaire les unes des autres.

42. Système à tuyau à retournement selon la revendication 41,
**caractérisé en ce que**
le tuyau à retournement (2), par le tronçon de tuyau intérieur (2a), les tronçons de tuyau extérieurs (2b, 2c), ainsi que la douille (5), constitue un espace creux, isolé de manière étanche par rapport à l'environnement, présentant un accès uniquement par les ouvertures (6) ménagées dans la douille (5).

43. Système à tuyau à retournement selon l'une des revendications 40 à 42,
**caractérisé en ce que**
la douille présente, sur sa face intérieure, une pluralité de rainures (5a) s'étendant sensiblement en direction axiale, ouvertures débouchant sur les faces frontales de la douille (5).

44. Système à tuyau à retournement selon la revendication 22,
**caractérisé par**
un dispositif de lubrification (15), pour l'introduction sous pression de lubrifiant dans un intervalle annulaire (9) situé entre le trocart ou la tige de trocart (1) et le tronçon de tuyau à retournement intérieur (2a), sachant qu'au moins une ouverture (17) ou une perforation, s'étendant sensiblement radialement, est prévue dans l'enveloppe extérieure (32) du trocart (1), découchant dans l'intervalle annulaire (9) et raccordé à un dispositif de transfert de lubrifiant (15).

45. Système à tuyau à retournement selon la revendication 44,
**caractérisé en ce que,**
entre l'enveloppe extérieure (32) et l'enveloppe intérieure (33) du trocart (1), est prévue au moins une conduite à lubrifiant, en tant que canal fonctionnel supplémentaire, conduite présentant des passages radiaux, situés selon des espacements déterminés en direction axiale, à travers l'enveloppe extérieure (33), pour assurer l'alimentation en lubrifiant du tuyau à retournement depuis la paroi extérieure du trocart (1) et la face intérieure du tuyau à retournement, sur toute la longueur du trocart (1), et susceptible d'être raccordée à un dispositif de transfert de lubrifiant.
